# EUROPEAN PATENT APPLICATION

(11) **EP 4 631 931 A1**
(43) Date of publication of application: **15.10.2025**
(21) Application number: 23900022.7
(22) Date of filing: 06.12.2023
(51) Int. Cl.: C07D 213/56, A61K 31/4406, A61P 35/00, A61P 35/02

(54) **HDAC INHIBITOR AND USE THEREOF**

(30) Priority: 07.12.2022 CN 202211570665; 13.06.2023 CN 202310697924
(71) Applicant: Wigen Biomedicine Technology (Shanghai) Co., Ltd., Shanghai 201203 (CN)
(72) Inventor: XU, Ruihua, Guangzhou, Guangdong 510062 (CN); WANG, Feng, Guangzhou, Guangdong 510095 (CN); XIE, Yuli, Shanghai 201203 (CN); LIU, Wenzhong, Shanghai 201203 (CN); QIAN, Lihui, Shanghai 201203 (CN)
(74) Representative: Dr. Langfinger & Partner
(86) International application number: PCT/CN2023/136827
(87) International publication number: WO 2024/120448

(57) **Abstract**

An HDAC inhibitor and the use thereof. Specifically, the present invention relates to a compound as represented by general formula (1) and a preparation method therefor, and the use of the compound of general formula (1) and each isomer, each crystal form, a pharmaceutically acceptable salt, hydrate or solvate thereof as an HDAC inhibitor in the preparation of an anti-tumor drug.

## Description

The present application claims priority to Chinese Patent Application No. 2022115706657 filed on December 7, 2022 and Chinese Patent Application No. 2023106979240 filed on June 13, 2023, which are incorporated herein by reference in their entirety.

### TECHNICAL FIELD

The present disclosure pertains to the field of pharmaceutical chemistry, and particularly relates to a class of HDAC inhibitors, a preparation method therefor, and use of the compound in preparing a medicament for treating or preventing cancer.

### BACKGROUND

Histone deacetylases (HDACs) are a class of proteases that regulate gene expression and protein function by deacetylating histone and non-histone lysine. There are currently four major classes of HDAC members, with 18 different subtypes. Class I includes four subtypes, HDAC1, HDAC2, HDAC3, and HDAC8; class II includes six subtypes, HDAC4, HDAC5, HDAC6, HDAC7, HDAC9, and HDAC10; class IV includes only one subtype, HDAC11. Class I, class II, and class IV are structurally homologous, while class III includes a total of 7 SIRT1-7 subtypes and shares no structural homology with the aforementioned three classes. HDAC inhibitors inhibit the growth and survival of a variety of tumor cells *in vitro.* Several HDAC inhibitors, represented by chidamide, have been approved for clinical use as monotherapy or in combination regimens for treating relapsed or refractory peripheral T-cell lymphoma, multiple myeloma, large B-cell lymphoma, and breast cancer.

Recently, studies have shown that in addition to functioning in tumor cells, specific HDAC subtypes have the function of regulating tumor immunity. For example, inhibition of HDAC1 and HDAC2 upregulates NKG2D expression, thereby enhancing the tumor-killing capacity of NK cells (Molecules, 2021, 26(13): 3952); inhibition of HDAC3 upregulates CXCL10-mediated immune cell infiltration (Cancer Immunol Res, 2023, 11(5): 657); inhibition of HDAC6 downregulates inflammasome-mediated IL-1β release (Int J Mol Med, 2024, 53(1): 1-14). HDAC10 modulates the function of NK cells by regulating the expression of CXCL10 (Proc Natl Acad Sci, 2021, 118(30): e2102718118). HDAC inhibitors can also function to regulate the tumor immune microenvironment and exert anti-tumor effects by suppressing angiogenesis. Inhibitors targeting various HDAC subtypes also exhibit different regulatory effects on other important immune cells such as T cells. Inhibitors targeting different HDAC subtypes can enhance the efficacy of tumor immunotherapeutic drugs such as immune checkpoint inhibitors anti-PD-1 monoclonal antibodies, and overcome drug resistance to tumor immunotherapeutic drugs. Therefore, the development of drugs with a selective inhibitory effect on specific HDAC subtypes can effectively enhance the therapeutic effect of immune checkpoint inhibitors, with great clinical value.

### SUMMARY

The present disclosure has unexpectedly discovered a class of unique HDAC inhibitors with selectivity toward specific subtypes and superior tumor immune synergy and metabolic stability. In addition, it is observed in *in vivo* studies that the compound of the present disclosure, when used in combination with a PD-1 inhibitor and a VEGF inhibitor, demonstrates a remarkable synergistic effect, and can cause tumor regression or even complete tumor disappearance, exhibiting strong anti-tumor efficacy.

The present disclosure provides a compound of general formula (1) or an isomer thereof, a crystalline form thereof, a pharmaceutically acceptable salt thereof, a hydrate thereof, or a solvate thereof: 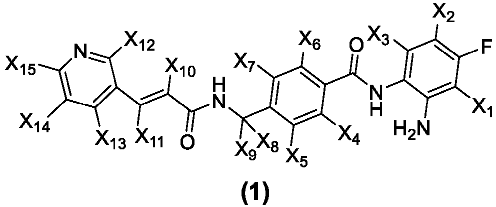 wherein in general formula (1):
X₁, X₂, X₃, X₄, X₅, X₆, X₇, X₈, X₉, X₁₀, X₁₁, X₁₂, X₁₃, X₁₄, and X₁₅ are each independently hydrogen or deuterium, and at least one of X₁, X₂, X₃, X₄, X₅, X₆, X₇, X₈, X₉, X₁₀, X₁₁, X₁₂, X₁₃, X₁₄, or X₁₅ is selected from deuterium.

In another preferred embodiment, in general formula (1), X₈ = X₉.

In another specific embodiment of the present disclosure, the compound of the present disclosure has one of the following structures:

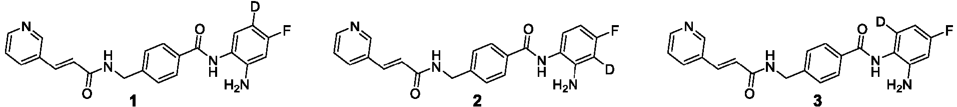

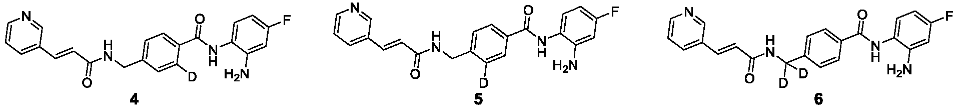

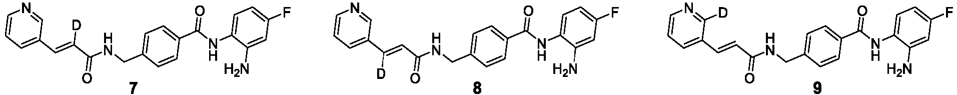

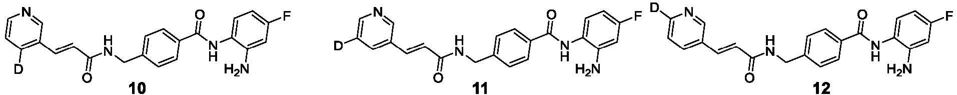

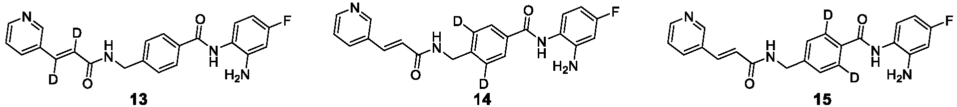

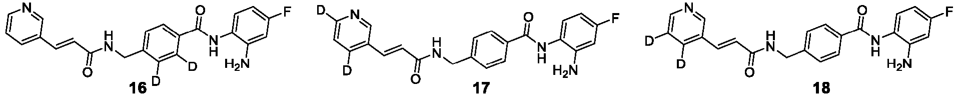

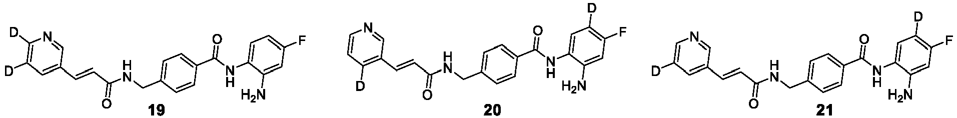

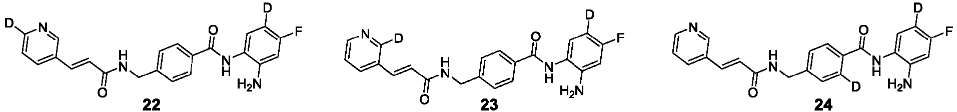

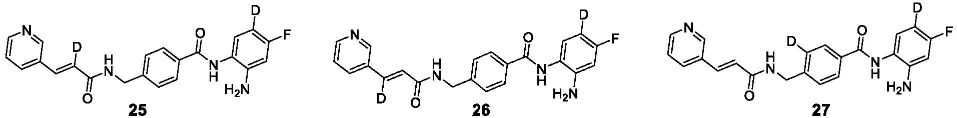

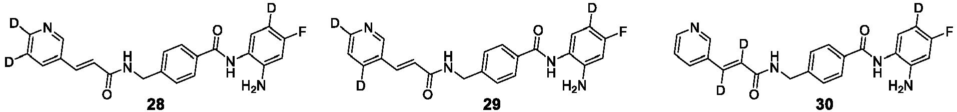

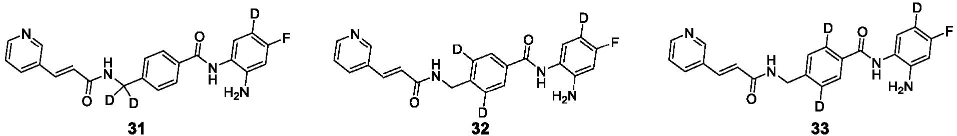

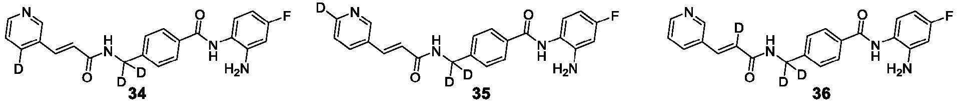

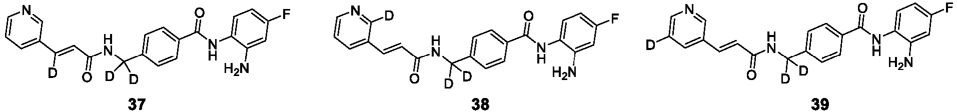

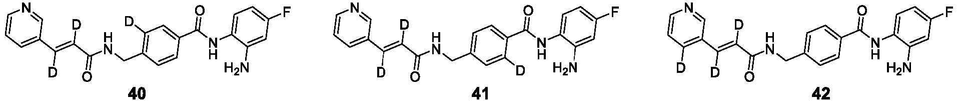

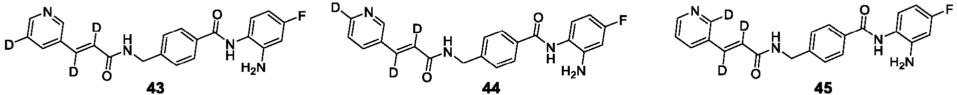

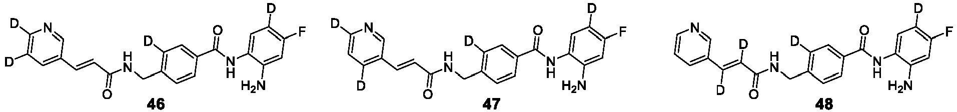

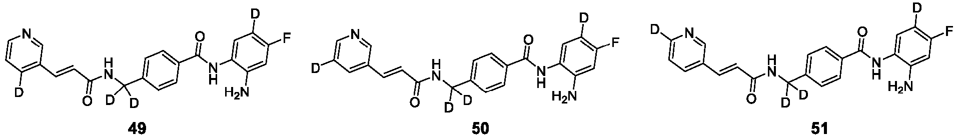

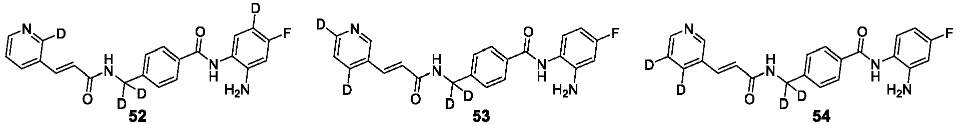

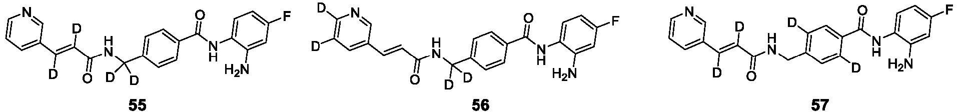

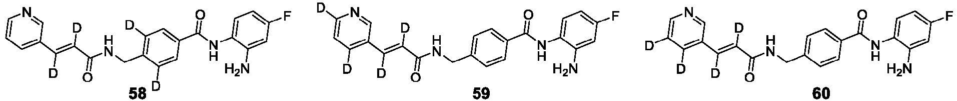

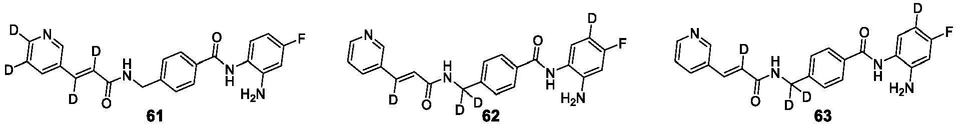

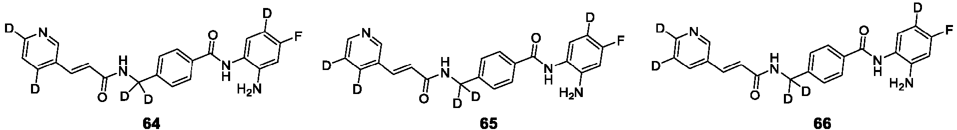

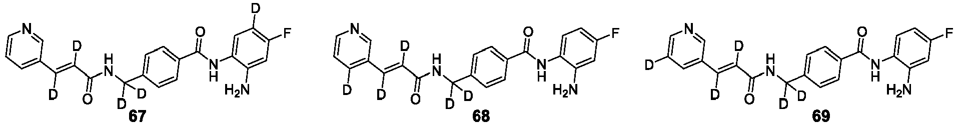

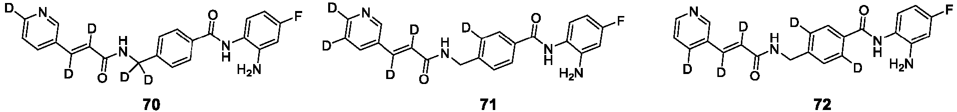

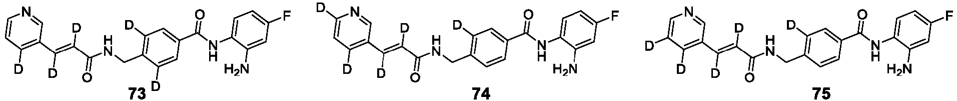

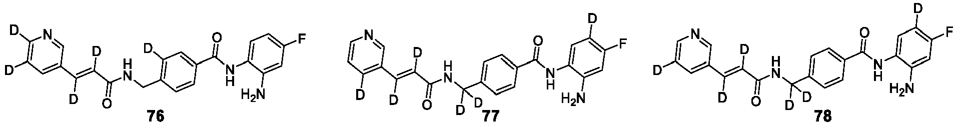

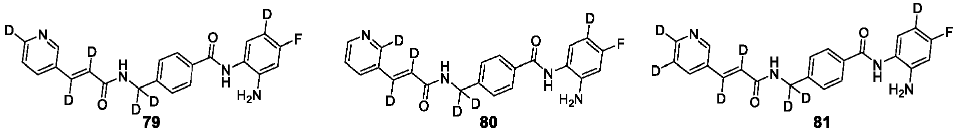

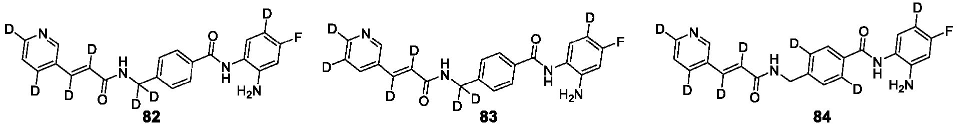

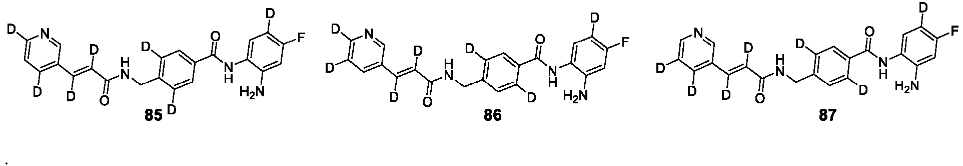

Another objective of the present disclosure is to provide a pharmaceutical composition comprising a pharmaceutically acceptable carrier, diluent, and/or excipient, as well as the compound of general formula (1) or the isomer thereof, the crystalline form thereof, the pharmaceutically acceptable salt thereof, the hydrate thereof, or the solvate thereof of the present disclosure as an active ingredient.

Still another objective of the present disclosure provides use of the compound of general formula (1) or the isomer thereof, the crystalline form thereof, the pharmaceutically acceptable salt thereof, the hydrate thereof, or the solvate thereof of the present disclosure, or the pharmaceutical composition described above in preparing a medicament for treating, regulating, or preventing a disease related to HDAC. wherein the disease is preferably cancer, and the cancer is a hematologic cancer or a solid tumor.

It should be understood that both the aforementioned general description and the following detailed description of the present disclosure are exemplary and explanatory, and are intended to provide further explanation of the present disclosure claimed.

### Synthesis of Compounds

Methods for preparing the compound of general formula (1) of the present disclosure are specifically described below, but these specific methods do not limit the present disclosure in any way.

The compound of general formula (1) described above may be synthesized using standard synthetic techniques or well-known techniques in combination with the methods described herein. In addition, the solvents, temperatures, and other reaction conditions mentioned herein may vary. Starting materials for the synthesis of the compounds may be obtained synthetically or commercially. The compounds described herein and other related compounds with different substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds may be altered by using appropriate reagents and conditions for introducing different groups into the molecular formulas provided herein. In one aspect, the compounds described herein are prepared according to methods well known in the art. However, the conditions of the methods, such as reactants, solvents, bases, the amount of the compound used, reaction temperature, and time required for the reaction are not limited to the following explanation. The compounds of the present disclosure may also be conveniently prepared by optionally combining various synthetic methods described herein or known in the art, and such combinations may be easily determined by those skilled in the art to which the present disclosure pertains. In one aspect, the present disclosure further provides a method for preparing the compound of general formula (1), which is prepared using general reaction scheme 1 below:

### General Reaction Scheme 1

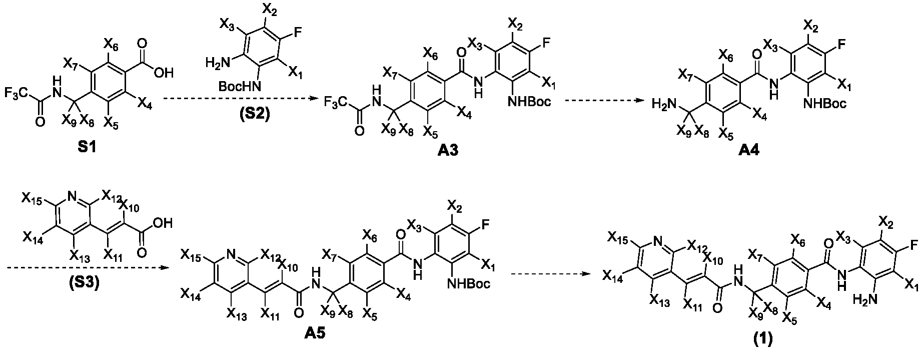

X₁, X₂, X₃, X₄, X₅, X₆, X₇, X₈, X₉, X₁₀, X₁₁, X₁₂, X₁₃, X₁₄, and X₁₅ are as defined above. As shown in general reaction scheme 1, starting materials S1 and S2 are subjected to an amide condensation reaction to give compound A3, A3 is deprotected under appropriate conditions to give compound A4, A4 is then subjected to a condensation reaction with S3 to give compound A5, and A5 is deprotected under acidic conditions to give the target compound (1).

### Further Forms of Compounds

"Pharmaceutically acceptable" herein refers to a substance, such as a carrier or diluent, which will not lead to loss of biological activity or properties of a compound and is relatively nontoxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed., (Wiley, 2002).

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol, or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized, and nanoparticle forms. In addition, the compound of general formula (1) includes crystalline forms, and may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability, and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer, and a cis-trans isomer. Each chiral center or axial chirality will independently produce two optical isomers, and all possible optical isomers, diastereomeric mixtures, and pure or partially pure compounds are included within the scope of the present disclosure. The present disclosure is meant to include all such isomeric forms of these compounds.

The compound of the present disclosure may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life, and the like. All isotopic variations of the compound of the present disclosure, whether radioactive or not, are contained within the scope of the present disclosure.

Any atom of the compound of the present disclosure, unless otherwise specified, refers to an isotope of the atom in stable state of the compound. Unless otherwise specified, when a site in a molecular structure is selected as "H" or "hydrogen", the site should be understood as having the natural abundance of the hydrogen isotope. Similarly, unless otherwise specified, when a site is selected as "D" or "deuterium", the site should be understood to have a deuterium isotopic abundance that is at least 3000 times its natural abundance (the natural abundance of the deuterium isotope is 0.015%).

More preferably, each deuterated site of the deuterated compounds of the present disclosure has a deuterium atom abundance that is at least 3500 times its natural abundance (52.2% deuterium atom enrichment). More preferably, the deuterium atom abundance is at least 4500 times the natural abundance (67.5% deuterium atom enrichment). More preferably, the deuterium atom abundance is at least 5000 times the natural abundance (75% deuterium atom enrichment). More preferably, the deuterium atom abundance is at least 6000 times the natural abundance (90% deuterium atom enrichment). More preferably, the deuterium atom abundance is at least 6333 times the natural abundance (95% deuterium atom enrichment). More preferably, the deuterium atom abundance is at least 6466.7 times the natural abundance (97% deuterium atom enrichment). More preferably, the deuterium atom abundance is at least 6600 times the natural abundance (99% deuterium atom enrichment). More preferably, the deuterium atom abundance is at least 6633.3 times the natural abundance (99.5% deuterium atom enrichment).

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques, and pharmacology are used. As used herein, "or" or "and" refers to "and/or" unless otherwise stated.

The term "isomer" refers to any tautomer, stereoisomer, atropisomer, isotopic isomer, enantiomer, or diastereomer of any compound of the present disclosure. The compound of the present disclosure may have one or more chiral centers or double bonds, and thus exists in the form of stereoisomers, e.g., double bond isomers (i.e., E/Z geometric isomers), or diastereomers (e.g., enantiomers (i.e., (+) or (-)) or cis/trans isomers). The compound of the present disclosure therefore encompasses all corresponding stereoisomers, i.e., stereoisomerically pure (e.g., geometrically pure, enantiomerically pure, or diastereomerically pure) forms, as well as mixtures of enantiomers and stereoisomers, e.g., racemates. The mixtures of enantiomers and stereoisomers of the compound of the present disclosure may be resolved into their component enantiomers or stereoisomers by well-known methods, such as chiral gas chromatography and chiral high-performance liquid chromatography, and by crystallization of the compound in the form of chiral salt complexes or in chiral solvents. Enantiomers and stereoisomers may also be obtained from stereomerically pure or enantiomerically pure intermediates, reagents, and catalysts by well-known asymmetric synthetic methods.

The term "isotopic isomer" refers to distinct molecules that differ in structure only by their isotopic composition and are identical in the remaining structure.

### Specific Pharmaceutical and Medical Terminology

The term "acceptable", as used herein, means that a formula component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign **caused** by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present disclosure may contain one or more asymmetric centers (chiral center or axial chirality) and thus occurs in the forms of a racemate, a racemic mixture, a single enantiomer, a diastereomeric compound, and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present disclosure. The present disclosure is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present disclosure are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present disclosure provides a method for treating a disease, including but not limited to a related condition involving an HDAC enzyme (e.g., cancer), with the compound of general formula (1) or the pharmaceutical compositions of the present disclosure.

In some embodiments, a method for treating cancer is provided, the method including administering to an individual in need thereof an effective amount of any of the aforementioned pharmaceutical compositions comprising the compound of general formula (1). In some embodiments, the cancer is mediated by a related HDAC enzyme. In some embodiments, the compound of the present disclosure is used in combination with an immune checkpoint inhibitor; in some embodiments, the compound of the present disclosure is used in combination with a PD-1 or PD-L1 inhibitor; in some embodiments, the compound of the present disclosure is used in combination with a PD-1 antibody; in some embodiments, the compound of the present disclosure is used in combination with a PD-L1 antibody; in some embodiments, the compound of the present disclosure is used in combination with a VEGF/VEGFR inhibitor; in some embodiments, the compound of the present disclosure is used in combination with an immune checkpoint inhibitor and a VEGF/VEGFR inhibitor; in some embodiments, the compound of the present disclosure is used in combination with a PD-1 inhibitor and a VEGF/VEGFR inhibitor; in some embodiments, the compound of the present disclosure is used in combination with a PD-1 antibody and a VEGF/VEGFR inhibitor; the PD-1 antibody includes, but is not limited to, nivolumab, pembrolizumab, toripalimab, sintilimab, camrelizumab, tislelizumab, penpulimab, zimberelimab, serplulimab, pucotenlimab, pidilizumab, cemiplimab, spartalizumab, AMG404, RN888, mAbl5, MEDI-0680, BGB-108, spartalizumab, IBI-308, mDX-400, SHR-1210, PF-06801591, PDR-001, GB-226, and STI-1110, and biosimilars, biobetters, and bioequivalents of these inhibitors; the PD-L1 antibody includes, but is not limited to, durvalumab, atezolizumab, envafolimab, sugemalimab, avelumab, avelumab, BMS-936559, AMP-714, ALN-PDL, TSR-042, KD-033, CA-170, STI-1014, and KY-1003, and biosimilars, biobetters, and bioequivalents of these inhibitors; the VEGF/VEGFR inhibitor includes, but is not limited to, bevacizumab, ranibizumab, ramucirumab, sorafenib, axitinib, apatinib, sunitinib, regorafenib, vandetanib, pazopanib, lenvatinib, cabozantinib, ponatinib, aflibercept, and fruquintinib. In other embodiments, the tumor includes, but is not limited to: breast cancer, colon cancer, uterine cancer, pancreatic cancer, lung cancer, gastric cancer, leukemia, lymphoma, prostate cancer, liver cancer, cervical cancer, neuroblastoma, melanoma, or intracranial tumors.

### Route of Administration

The compound and the pharmaceutically acceptable salt thereof of the present disclosure can be made into various formulations comprising a safe and effective amount of the compound or the pharmaceutically acceptable salt thereof of the present disclosure, and a pharmaceutically acceptable excipient or carrier. The "safe and effective amount" means that the amount of the compound is sufficient to significantly improve the condition without causing serious side effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present disclosure and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present disclosure is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills, and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents. In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions, or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present disclosure include ointments, pulvises, patches, sprays, and inhalants. The active ingredient is mixed under sterile conditions with a physiologically acceptable carrier and any preservatives, buffers, or propellants that may be required if necessary.

The compound of the present disclosure may be administered alone or in combination with other pharmaceutically acceptable compounds. When the pharmaceutical composition is used, a safe and effective amount of the compound of the present disclosure is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient, and the like will also be considered, which are well-known to skilled physicians.

The above features mentioned in the present disclosure or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present disclosure very clear. It should be understood that the detailed description and examples below describe specific examples for reference only. After reading the description of the present disclosure, those skilled in the art can make various changes or modifications to the present disclosure, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present disclosure: ACN represents acetonitrile; AcOH represents glacial acetic acid; AIBN represents azobisisobutyronitrile; Boc₂O represents di-tert-butyl dicarbonate; CDCl₃ represents deuterated chloroform; (COCl)₂ represents oxalyl chloride; D₂ represents deuterium gas; D₂O represents heavy water; DBU represents 1,8-diazabicyclo[5.4.0]undec-7-ene; DCM represents dichloromethane; Dioxane represents 1,4-dioxane; DIPEA represents diisopropylethylamine; DMSO represents dimethyl sulfoxide; DMAP represents 4-dimethylaminopyridine; DMF represents N,N-dimethylformamide; EA represents ethyl acetate; EtOH represents ethanol; EDCI represents 1-(3-dimethylaminopropyl)-3-ethylcarbodiimide hydrochloride; FA represents formic acid; Flash represents flash preparative medium-pressure liquid chromatography; h represents hour; H₂ represents hydrogen; HOBt represents 1-hydroxybenzotriazole; K₂CO₃ represents anhydrous potassium carbonate; KOH represents potassium hydroxide; LC-MS represents liquid chromatography-mass spectrometry; LiOH represents lithium hydroxide; LiOH.H₂O represents lithium hydroxide monohydrate; MeOH represents absolute methanol; MeOD represents monodeuteriomethanol; min represents minute; mL represents milliliter; MS represents mass spectrometry; NaBD₄ represents sodium borodeuteride; NaOAc represents anhydrous sodium acetate; n-BuLi represents n-butyl aluminum; NaBH(OAc)₃ represents sodium triacetoxyborohydride; NBS represents N-bromosuccinimide; NH₄Cl represents ammonium chloride; NMR represents nuclear magnetic resonance; Pd/C represents palladium on carbon; PE represents petroleum ether; PPTS represents pyridinium 4-toluenesulfonate; TFA represents trifluoroacetic acid; TFAArepresents trifluoroacetic anhydride; THF represents tetrahydrofuran; Zn represents zinc powder.

### Preparation Example 1. Synthesis of 4-((2,2,2-trifluoroacetamido)methyl)benzoic-2-d acid (S1-1)

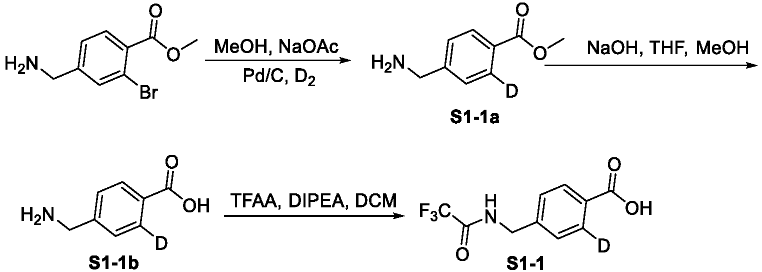

### Synthesis of S1-1a:

To a 100 mL single-neck flask were added methyl 2-bromo-4-(aminomethyl)benzoate (2.0 g, 8.2 mmol), sodium acetate (1 g), 10% Pd/C (200 mg), and MeOH (40 mL). The system was purged three times with deuterium gas, and then stirred at room temperature for 6 h under an atmosphere of deuterium supplied via a balloon. After the completion of the reaction as detected by LC-MS, the mixed solution was filtered, and the filtrate was concentrated under reduced pressure until a small amount remained. EA (100 mL) and a saturated sodium bicarbonate solution (50 mL) were added to the residue, and the mixture was stirred and subjected to liquid separation. The organic phase was then washed twice with saturated sodium chloride, dried over anhydrous sodium sulfate, filtered, and concentrated to dryness to give a colorless oily product (1.82 g, >100%).

ESI-MS m/z: 167.1 [M+H]⁺.

### Synthesis of S1-1b:

To a 100 mL single-neck flask were added the compound **S1-1a** described above (1.82 g, crude, 8.2 mmol), THF (20 mL), MeOH (10 mL), and NaOH (1.6 g, 40.0 mmol). The mixed solution was stirred at room temperature for 5 h. After the completion of the reaction as detected by LC-MS, the mixed solution was concentrated, and the residue was purified by Flash and lyophilized to give a white solid product (840 mg, 67.4%).

ESI-MS m/z: 153.0 [M+H]⁺.

### Synthesis of S1-1:

To a 250 mL single-neck flask were added the compound **S1-1b** described above (840 mg, 5.52 mmol), DCM (84 mL), and DIPEA (1.78 g, 13.8 mmol), and a solution of TFAA (1.74 g, 8.28 mmol) in DCM (10 mL) was added dropwise under an ice bath. After the completion of the dropwise addition, the mixture was stirred at room temperature for 6 h. After the completion of the reaction as detected by LC-MS, a 2 N HCl solution (30 mL) was added to the mixed solution. The mixture was stirred for 20 min and then subjected to liquid separation. The organic phase was then washed twice with saturated sodium chloride, dried over anhydrous sodium sulfate, filtered, and concentrated. The residue was slurried with EA (5 mL)/PE (30 mL) at room temperature for 30 min, filtered, and dried to give an off-white solid product (1.21 g, 88.4%).

ESI-MS m/z: 249.0 [M+H]⁺.

Target intermediates S1-2 to S1-6 were obtained using different starting materials according to the synthesis method for the intermediate S1-1.

**Table 1. Structural formulas of intermediates S1-2 to S1-6**

| **Intermediate** | **Compound structure** | **MS [M+H]⁺** | **Intermediate** | **Compound structure** | **MS [M+H]⁺** |
|---|---|---|---|---|---|
| **S1-2** | 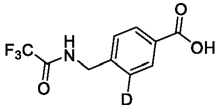 | 249.0 | **S1-3** | 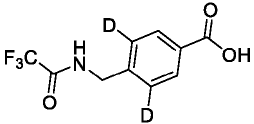 | 250.0 |
| **S1-4** | 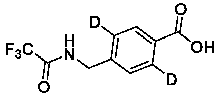 | 250.0 | **S1-5** | 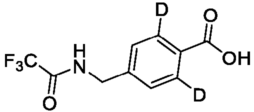 | 250.0 |
| **S1-6** | 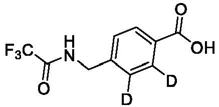 | 250.0 | | | |

### Preparation Example 2. Synthesis of 4-((2,2,2-trifluoroacetamido-d)methyl-d2)benzoic acid (S1-7)

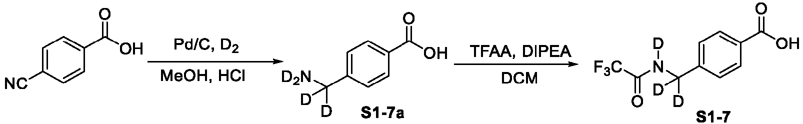

### Synthesis of S1-7a:

To a 500 mL single-neck flask were added 4-cyano-benzoic acid (2.94 g, 20.0 mmol), 4 M HCl/MeOH (5 mL, 20.0 mmol), and 10% Pd/C (300 mg) in MeOH (100 mL). The system was purged three times with deuterium gas, and then stirred at room temperature for 20 h under an atmosphere of deuterium supplied via a balloon. After the completion of the reaction as detected by LC-MS, the mixed solution was filtered, and the filtrate was concentrated to dryness under reduced pressure to give a white solid product (4.1 g, >100%).

ESI-MS m/z: 156.1 [M+H]⁺.

### Synthesis of S1-7:

To a 500 mL single-neck flask were added the compound S1-1b described above (4.1 g, 20.0 mmol), DCM (160 mL), and DIPEA (12.9 g, 0.1 mol), and a solution of TFAA (6.3 g, 30 mmol) in DCM (20 mL) was added dropwise under an ice bath. After the completion of the dropwise addition, the mixture was stirred at room temperature for 6 h. After the completion of the reaction as detected by LC-MS, a 2 N HCl solution (30 mL) was added to the mixed solution. The mixture was stirred for 20 min and then subjected to liquid separation. The organic phase was then washed twice with saturated sodium chloride, dried over anhydrous sodium sulfate, filtered, and concentrated. The residue was slurried with EA (20 mL)/PE (100 mL) at room temperature for 30 min, filtered, and dried to give an off-white solid product (2.65 g, 53%).

ESI-MS m/z: 251.0 [M+H]⁺.

### Preparation Example 3. Synthesis of tert-butyl (2-amino-5-fluorophenyl-4-d)carbamate (S2-1)

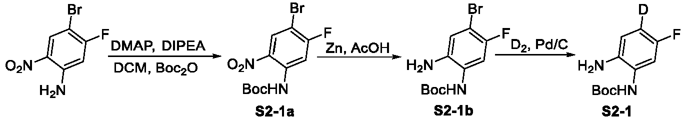

### Synthesis of S2-1a:

5-Fluoro-4-bromo-2-nitroaniline (2.35 g, 10.0 mmol) was dissolved in DCM (30 mL), and DMAP (610 mg, 5.0 mmol), DIPEA(2.58 g, 20.0 mmol), and Boc₂O (2.62 g, 12.0 mmol) were added. The mixture was heated at reflux for 20 h under an argon atmosphere. After the completion of the reaction as detected by LC-MS, water (50 mL) and DCM (50 mL) were added to the mixture. The resulting mixture was stirred and subjected to liquid separation. The organic phase was then washed with 1 N HCl (50 mL), washed with saturated sodium chloride, dried over anhydrous sodium sulfate, filtered, and concentrated to dryness to give a yellowish brown solid product (3.7 g, yield: >100%).

ESI-MS m/z: 335.0 [M+H]⁺.

### Synthesis of S2-1b:

The crude product **S2-1a** described above was added to THF (50 mL), and zinc powder (6.5 g, 0.1 mol) was then added. A solution of AcOH (6 g, 0.1 mol) in THF (10 mL) was slowly added dropwise under an ice bath. After the completion of the dropwise addition, the mixed solution was stirred at room temperature for 4 h. After the completion of the reaction as detected by LC-MS, the mixed solution was filtered. The filter cake was rinsed with THF, and the filtrate was concentrated. The residue was purified by column chromatography to give a brown solid product (1.52 g, 49.8%).

ESI-MS m/z: 305.0 [M+H]⁺.

### Synthesis of S2-1:

The compound **S2-1b** described above (1.43 g, 4.69 mmol), 10% Pd/C (100 mg), and sodium acetate (500 mg) were added to MeOH (30 mL). The system was purged three times with deuterium gas, and then stirred at room temperature for 20 h under an atmosphere of deuterium supplied via a balloon. After the completion of the reaction as detected by LC-MS, the mixed solution was filtered, and the filtrate was concentrated under reduced pressure until a small amount remained. EA (50 mL) and a saturated sodium bicarbonate solution (50 mL) were added to the residue, and the mixture was stirred and subjected to liquid separation. The organic phase was then washed twice with saturated sodium chloride, dried over anhydrous sodium sulfate, filtered, and concentrated to dryness to give a brown solid product (970 mg, 91.1%).

ESI-MS m/z: 228.1 [M+H]⁺.

Target intermediates S2-2 and S2-3 were obtained using different starting materials according to the synthesis method for the intermediate S2-1.

**Table 2. Structural formulas of intermediates S2-2 to S2-3**

| **Intermediate** | **Compound structure** | **MS [M+H]⁺** | **Intermediate** | **Compound structure** | **MS [M+H]⁺** |
|---|---|---|---|---|---|
| **S2-2** | 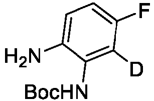 | 228.1 | **S2-3** | 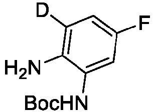 | 228.1 |

### Preparation Example 4. Synthesis of (E)-3-(pyridin-3-yl-4-d)acrylic acid (S3-1)

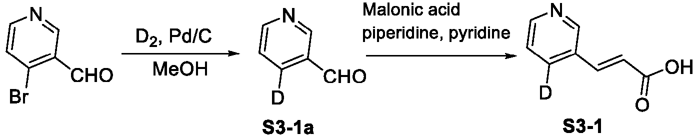

### Synthesis of S3-1a:

4-Bromopyridine-3-carbaldehyde (1.86 g, 10.0 mmol), 10% Pd/C (200 mg), and sodium acetate (1 g) were added to MeOH (30 mL). The system was purged three times with deuterium gas, and then stirred at room temperature for 20 h under an atmosphere of deuterium supplied via a balloon. After the completion of the reaction as detected by LC-MS, the mixed solution was filtered, and the filtrate was concentrated under reduced pressure until a small amount remained. EA (50 mL) and a saturated sodium bicarbonate solution (50 mL) were added to the residue, and the mixture was stirred and subjected to liquid separation. The organic phase was then washed twice with saturated sodium chloride, dried over anhydrous sodium sulfate, filtered, and concentrated to dryness to give a white solid product (1.1 g, >100%).

ESI-MS m/z: 109.1 [M+H]⁺.

### Synthesis of S3-1:

To a 100 mL single-neck flask were added the compound **S3-1a** described above (1.1 g, 10.0 mmol), malonic acid (2.08 g, 20 mmol), piperidine (2.55 g, 30 mmol), and pyridine (10 mL). The mixed solution was purged with argon, heated at reflux, and stirred for 20 h. After the completion of the reaction as detected by LC-MS, the mixed solution was concentrated, and the residue was purified by Flash and lyophilized to give an off-white solid product (853 mg, 56.9%).

ESI-MS m/z: 151.0 [M+H]⁺.

Target intermediates S3-2 to S3-7 were obtained using different starting materials according to the synthesis method for the intermediate S3-1.

**Table 3. Structural formulas of intermediates S3-2 to S3-7**

| **Intermediate** | **Compound structure** | **MS [M+H]⁺** | **Intermediate** | **Compound structure** | **MS [M+H]⁺** |
|---|---|---|---|---|---|
| **S3-2** | 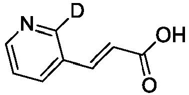 | 151.0 | **S3-3** | 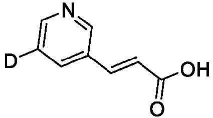 | 151.0 |
| **S3-4** | 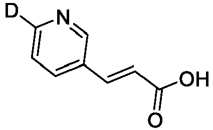 | 151.0 | **S3-5** | 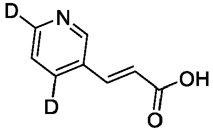 | 152.0 |
| **S3-6** | 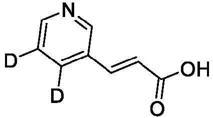 | 152.0 | **S3-7** | 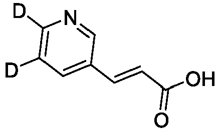 | 152.0 |

### Preparation Example 5. Synthesis of (E)-3-(pyridin-3-yl)acrylic-2,3-d2 acid (S3-8)

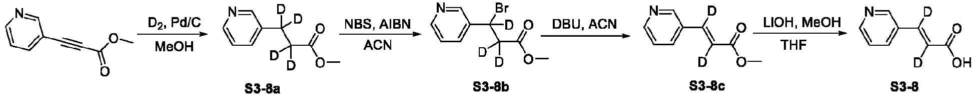

### Synthesis of S3-8a:

Methyl 3-(pyridin-3-yl)propiolate (1.61 g, 10.0 mmol) and 10% Pd/C (200 mg) were added to MeOH (30 mL). The system was purged three times with deuterium gas, and then stirred at room temperature for 20 h under an atmosphere of deuterium supplied via a balloon. After the completion of the reaction as detected by LC-MS, the mixed solution was filtered, and the filtrate was concentrated to dryness under reduced pressure to give an anhydrous oily product (1.8 g, >100%).

ESI-MS m/z: 170.1 [M+H]⁺.

### Synthesis of S3-8b:

To a 100 mL single-neck flask were added the compound **S3-8a** described above (1.8 g, 10.0 mmol), AIBN (164 mg, 1.0 mmol), and acetonitrile (40 mL), and NBS (1.96 g, 11.0 mmol) was then added in batches. The mixed solution was heated at reflux for 6 h. After the completion of the reaction as detected by LC-MS, the mixture was cooled and concentrated, and the residue was directly used in the next step.

### Synthesis of S3-8c:

To a 100 mL single-neck flask were added the compound **S3-8b** described above (5.2 g, crude, 10.0 mmol), DBU (3.04 g, 20.0 mmol), and acetonitrile (40 mL), and the mixture was then heated to 50 °C and reacted for 4 h. After the completion of the reaction as detected by LC-MS, the mixture was cooled and concentrated, and the residue was purified by Flash to give a colorless oily product (930 mg, 56.4%).

ESI-MS m/z: 166.1 [M+H]⁺.

### Synthesis of S3-8:

To a 100 mL single-neck flask were added the compound **S3-8c** described above (930 mg, 5.64 mmol), THF (5 mL), MeOH (5 mL), and LiOH.H₂O (474 mg, 11.3 mmol). The mixed solution was stirred at room temperature for 5 h. After the completion of the reaction as detected by LC-MS, the mixed solution was concentrated, and the residue was purified by Flash and lyophilized to give a white solid product (716 mg, 83.5%).

ESI-MS m/z: 152.0 [M+H]⁺.

Target intermediates S3-9 to S3-15 were obtained using different starting materials according to the synthesis method for the intermediate S3-8.

**Table 4. Structural formulas of intermediates S3-9 to S3-15**

| **Intermediate** | **Compound structure** | **MS [M+H]⁺** | **Intermediate** | **Compound structure** | **MS [M+H]⁺** |
|---|---|---|---|---|---|
| **S3-9** | 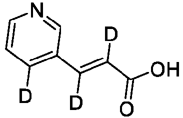 | 153.0 | **S3-10** | 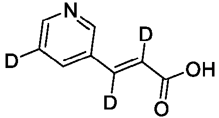 | 153.0 |
| **S3-11** | 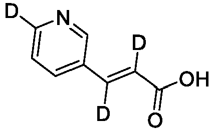 | 153.0 | **S3-12** | 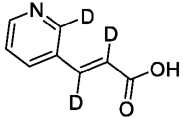 | 153.0 |
| **S3-13** | 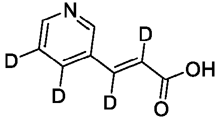 | 154.0 | **S3-14** | 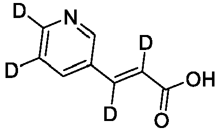 | 154.0 |
| **S3-15** | 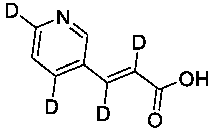 | 154.0 | | | |

### Preparation Example 6. Synthesis of (E)-3-(pyridin-3-yl)acrylic-3-d acid (S3-16)

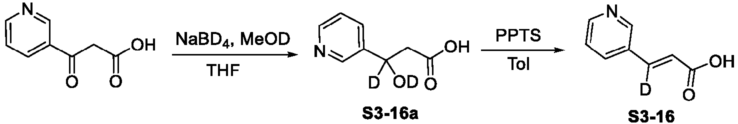

### Synthesis of S3-16a:

To a 100 mL single-neck flask were added 3-oxo-3-(pyridin-3-yl)propionic acid (2 g, 12.12 mmol), THF (30 mL), and MeOD (2 g), and NaBD₄ (509 mg, 12.12 mmol) was added in batches at room temperature. After the completion of the addition, the mixed solution was stirred at room temperature for 2 h. After the completion of the reaction as detected by LC-MS, D₂O (5 mL) was added dropwise to the mixed solution to quench the reaction. The mixture was then concentrated, and the residue was purified by Flash to give a colorless solid product (1.66 g, 81%).

ESI-MS m/z: 170.0 [M+H]⁺.

### Synthesis of S3-16:

To a 100 mL single-neck flask were added the compound **S3-16a** described above (1.66 g, 9.82 mmol), toluene (20 mL), PPTS (250 mg, 1.0 mmol), and 4 A molecular sieves (5 g), and the mixed solution was heated to 100 °C and stirred for 16 h. After the completion of the reaction as detected by LC-MS, the mixed solution was filtered. The filter cake was rinsed with THF, and the filtrate was concentrated to dryness. The residue was purified by Flash to give a white solid product (1.03 g, 69.9%).

ESI-MS m/z: 151.0 [M+H]⁺.

Target intermediate S3-17 was obtained using different starting materials according to the synthesis method for the intermediate S3-16.

**Table 5. Structural formula of intermediate S3-17**

| **Intermediate** | **Compound structure** | **MS [M+H]⁺** |
|---|---|---|
| **S3-17** | 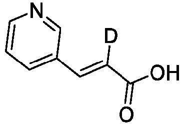 | 151.0 |

### Example 1. Synthesis of (E)-N-(2-amino-4-fluorophenyl-5-d)-4-((3-(pyridin-3-yl)acrylamido)methyl)benzamide (Compound 1)

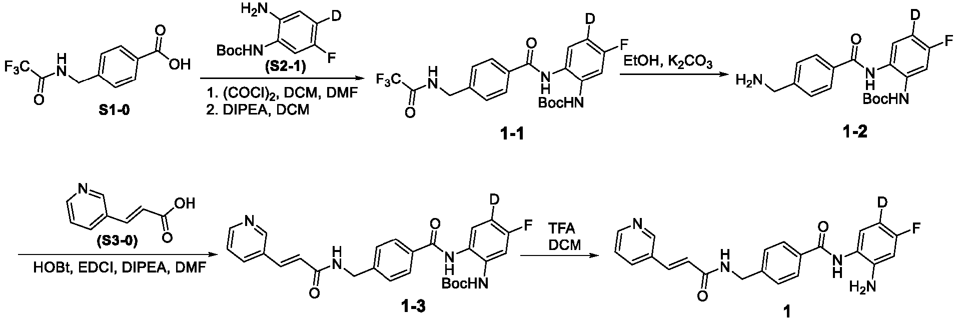

### Step 1: Synthesis of compound 1-1:

To a 100 mL single-neck flask were added 4-((2,2,2-trifluoroacetamido)methyl)benzoic acid (S1-0, 446 mg, 1.8 mmol), DMF (20 mg), and DCM (10 mL), and a solution of (COCl)₂ (343 mg, 2.7 mmol) in DCM (2 mL) was added dropwise at room temperature under an argon atmosphere. After the completion of the dropwise addition, the mixed solution was stirred at room temperature for 2 h. After the completion of the reaction as detected by LC-MS, the mixed solution was concentrated to dryness under pressure. DCM (10 mL) was then added to the residue, and then a solution of DIPEA (700 mg, 5.43 mmol) and **S2-1** (410 mg, 1.81 mmol) in DCM (5 mL) was sequentially added dropwise at room temperature. After the completion of the dropwise addition, the mixed solution was stirred at room temperature for 1 h. After the completion of the reaction as detected by LC-MS, DCM (30 mL) and water (30 mL) were added to the mixed solution. The mixture was stirred and then subjected to liquid separation. The organic phase was washed twice with a saturated sodium chloride solution, dried over anhydrous sodium sulfate, filtered, and concentrated under reduced pressure. The residue was then slurried with EA (2 mL)/PE (8 mL) at room temperature, filtered, and dried to give a light brown solid product (581 mg, 70.8%).

ESI-MS m/z: 457.1 [M+H]⁺.

### Step 2: Synthesis of compound 1-2:

To a 100 mL single-neck flask were added the compound **1-1** described above (581 mg, 1.27 mmol), MeOH (10 mL), and anhydrous potassium carbonate (351 mg, 2.54 mmol). The mixed solution was purged with argon, and then heated to 60 °C and stirred for 6 h. After the completion of the reaction as detected by LC-MS, the mixed solution was concentrated. DCM (30 mL) and a saturated sodium chloride solution (20 mL) were added to the resulting residue. The mixture was stirred and subjected to liquid separation. The organic phase was then washed with a saturated sodium chloride solution, dried over anhydrous sodium sulfate, filtered, and concentrated to dryness to give an off-white solid product (486 mg, 106%).

ESI-MS m/z: 361.1 [M+H]⁺.

### Step 3: Synthesis of compound 1-3:

To a 100 mL single-neck flask were added (E)-3-(pyridin-3-yl)acrylic acid **(S3-0,** 42 mg, 0.278 mmol), DIPEA(72 mg, 0.556 mmol), HOBt (56 mg, 0.417 mmol), EDCI (80 mg, 0.417 mmol), and DMF (5 mL). The mixed solution was stirred at room temperature for 15 min, and the compound **1-2** described above (106 mg, 0.278 mmol) was then added. The resulting mixture was then stirred for another 16 h. After the completion of the reaction as detected by LC-MS, the mixture was purified by Flash to give a yellowish solid product (118 mg, 86.4%).

ESI-MS m/z: 492.1 [M+H]⁺.

### Step 4: Synthesis of compound 1:

To a 100 mL single-neck flask were added the compound **1-3** described above (118 mg, 0.24 mmol), DCM (5 mL), and TFA (0.5 mL), and the mixed solution was stirred at room temperature for 2 h. After the completion of the reaction as detected by LC-MS, the mixture was concentrated, and the residue was purified by preparative liquid chromatography to give a white solid product (60 mg, 64%).

ESI-MS m/z: 392.1 [M+H]⁺.

### Example 2. Synthesis of (E)-N-(2-amino-4-fluorophenyl)-4-((3-(pyridin-3-yl)acrylamido)methyl)-3-d benzamide (Compound 5)

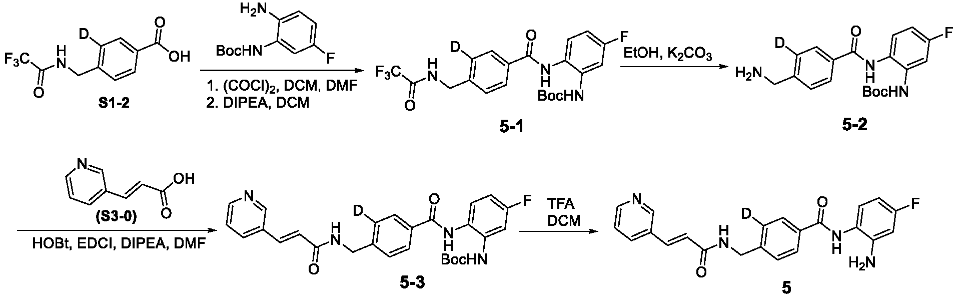

### Step 1: Synthesis of compound 5-1:

To a 100 mL single-neck flask were added 4-((2,2,2-trifluoroacetamido)methyl)-3-d-benzoic acid (S1-2, 600 mg, 2.42 mmol), DMF (30 mg), and DCM (10 mL), and a solution of (COCl)₂ (461 mg, 3.63 mmol) in DCM (2 mL) was added dropwise at room temperature under an argon atmosphere. After the completion of the dropwise addition, the mixed solution was stirred at room temperature for 2 h. After the completion of the reaction as detected by LC-MS, the mixed solution was concentrated to dryness under pressure. DCM (10 mL) was then added to the residue, and then a solution of DIPEA (1.03 g, 8.0 mmol) and tert-butyl (2-amino-5-fluorophenyl)carbamate (547 mg, 2.42 mmol) in DCM (5 mL) was sequentially added dropwise at room temperature. After the completion of the dropwise addition, the mixed solution was stirred at room temperature for 1 h. After the completion of the reaction as detected by LC-MS, DCM (30 mL) and water (30 mL) were added to the mixed solution. The mixture was stirred and then subjected to liquid separation. The organic phase was washed twice with a saturated sodium chloride solution, dried over anhydrous sodium sulfate, filtered, and concentrated under reduced pressure. The residue was then slurried with EA (2 mL)/PE (8 mL) at room temperature, filtered, and dried to give a light brown solid product (853 mg, 77.3%). ESI-MS m/z: 457.1 [M+H]⁺.

### Step 2: Synthesis of compound 5-2:

To a 100 mL single-neck flask were added the compound 5-1 described above (853 mg, 1.87 mmol), MeOH (10 mL), and anhydrous potassium carbonate (516 mg, 3.74 mmol). The mixed solution was purged with argon, and then heated to 60 °C and stirred for 6 h. After the completion of the reaction as detected by LC-MS, the mixed solution was concentrated. DCM (30 mL) and a saturated sodium chloride solution (20 mL) were added to the resulting residue. The mixture was stirred and subjected to liquid separation. The organic phase was then washed with a saturated sodium chloride solution, dried over anhydrous sodium sulfate, filtered, and concentrated to dryness to give an off-white solid product (660 mg, 98.0%).

ESI-MS m/z: 361.1 [M+H]⁺.

### Step 3: Synthesis of compound 5-3:

To a 100 mL single-neck flask were added (E)-3-(pyridin-3-yl)acrylic acid **(S3-0,** 63 mg, 0.417 mmol), DIPEA (108 mg, 0.837 mmol), HOBt (84 mg, 0.622 mmol), EDCI (120 mg, 0.626 mmol), and DMF (10 mL). The mixed solution was stirred at room temperature for 15 min, and the compound **5-2** described above (160 mg, 0.417 mmol) was then added. The resulting mixture was then stirred for another 16 h. After the completion of the reaction as detected by LC-MS, the mixture was purified by Flash to give a yellowish solid product (140 mg, 68.3%).

ESI-MS m/z: 492.1 [M+H]⁺.

¹H NMR (600 MHz, DMSO-d₆) δ 9.77 (s, 1H), 8.84 - 8.72 (m, 3H), 8.56 (dd, *J= 4.7,* 1.7 Hz, 1H), 8.01 (dt, *J =* 8.0, 2.0 Hz, 1H), 7.97 - 7.90 (m, 2H), 7.53 (dd, *J =* 15.0, 5.4 Hz, 2H), 7.46 (dt, *J =* 8.4, 2.1 Hz, 3H), 6.97 (td, *J =* 8.4, 3.0 Hz, 1H), 6.83 (d, *J =* 15.9 Hz, 1H), 4.51 (d, *J* = 6.0 Hz, 2H), 1.45 (s, 9H).

### Step 4: Synthesis of compound 5:

To a 100 mL single-neck flask were added the compound **5-3** described above (140 mg, 0.285 mmol), DCM (5 mL), and TFA (0.5 mL), and the mixed solution was stirred at room temperature for 2 h. After the completion of the reaction as detected by LC-MS, the mixture was concentrated, and the residue was purified by preparative liquid chromatography to give a white solid product (82 mg, 73.5%).

ESI-MS m/z: 392.1 [M+H]⁺.

By the procedures similar to those in the synthesis of **compound 1** and **compound** 5, other target compounds in Table 6 can be obtained using different intermediates as starting materials.

**Table 6**

| **Compound** | **Structural formula** | **MS: [M+H]⁺** | **Compound** | **Structural formula** | **MS: [M+H]⁺** |
|---|---|---|---|---|---|
| **2** | 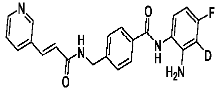 | 392.1 | **3** | 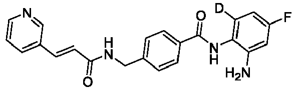 | 392.1 |
| **4** | 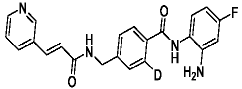 | 392.1 | **5** | 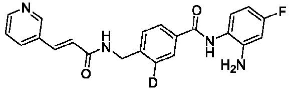 | 392.1 |
| **6** | 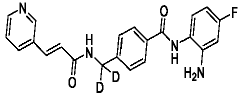 | 393.1 | **7** | 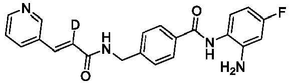 | 392.1 |
| **8** | 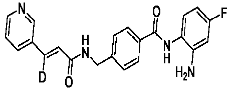 | 392.1 | **9** | 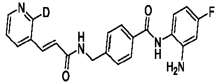 | 392.1 |
| **10** | 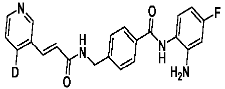 | 392.1 | **11** | 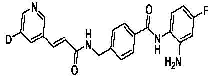 | 392.1 |
| **12** | 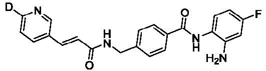 | 392.1 | **13** | 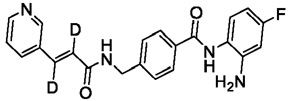 | 393.1 |
| **14** | 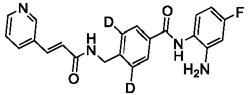 | 393.1 | **15** | 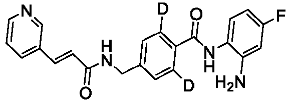 | 393.1 |
| **16** | 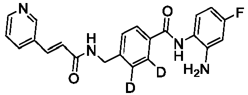 | 393.1 | **17** | 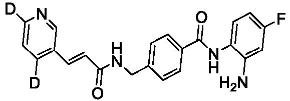 | 393.1 |
| **18** | 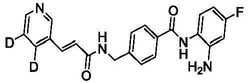 | 393.1 | **19** | 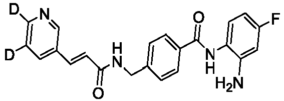 | 393.1 |
| **20** | 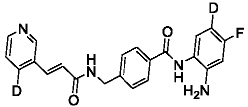 | 393.1 | **21** | 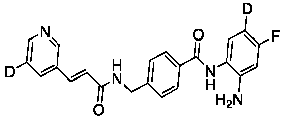 | 393.1 |
| **22** | 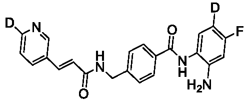 | 393.1 | **23** | 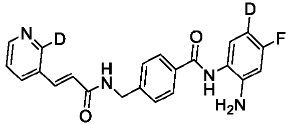 | 393.1 |
| **24** | 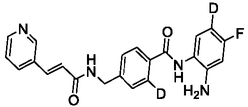 | 393.1 | **25** | 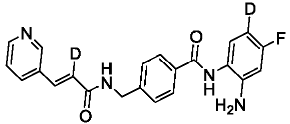 | 393.1 |
| **26** | 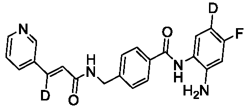 | 393.1 | **27** | 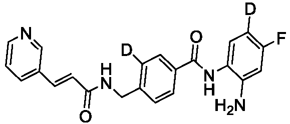 | 393.1 |
| **28** | 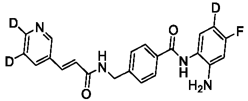 | 394.1 | **29** | 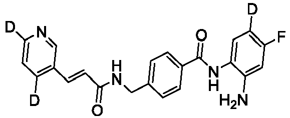 | 394.1 |
| **30** | 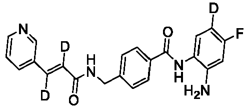 | 394.1 | **31** | 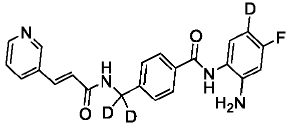 | 394.1 |
| **32** | 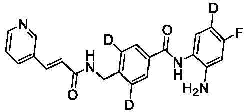 | 394.1 | **33** | 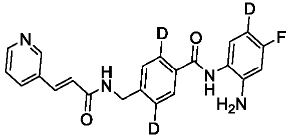 | 394.1 |
| **34** | 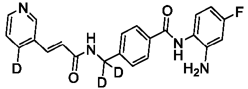 | 394.1 | **35** | 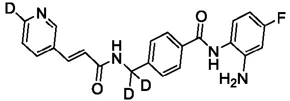 | 394.1 |
| **36** | 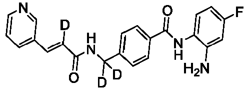 | 394.1 | **37** | 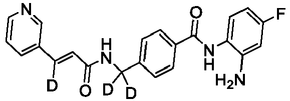 | 394.1 |
| **38** | 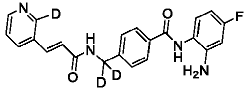 | 394.1 | **39** | 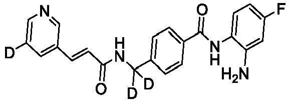 | 394.1 |
| **40** | 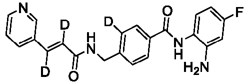 | 394.1 | **41** | 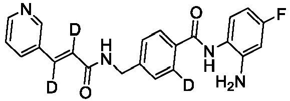 | 394.1 |
| **42** | 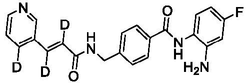 | 394.1 | **43** | 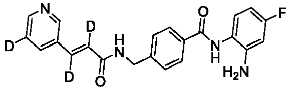 | 394.1 |
| **44** | 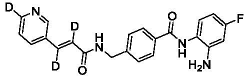 | 394.1 | **45** | 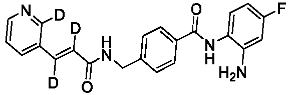 | 394.1 |
| **46** | 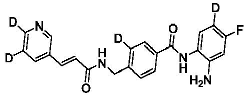 | 395.1 | **47** | 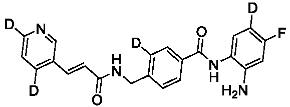 | 395.1 |
| **48** | 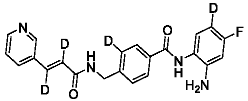 | 395.1 | **49** | 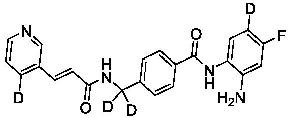 | 395.1 |
| **50** | 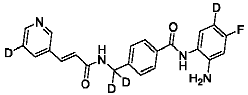 | 395.1 | **51** | 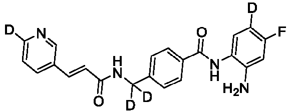 | 395.1 |
| **52** | 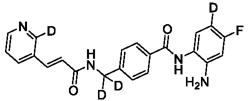 | 395.1 | **53** | 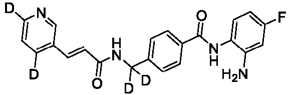 | 395.1 |
| **54** | 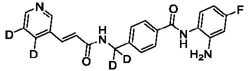 | 395.1 | **55** | 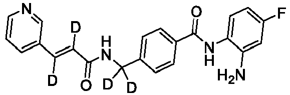 | 395.1 |
| **56** | 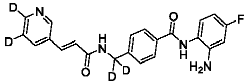 | 395.1 | **57** | 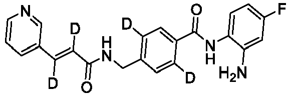 | 395.1 |
| **58** | 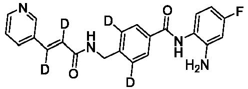 | 395.1 | **59** | 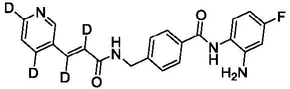 | 395.1 |
| **60** | 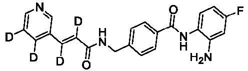 | 395.1 | **61** | 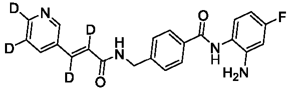 | 395.1 |
| **62** | 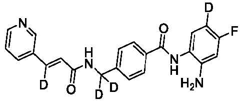 | 395.1 | **63** | 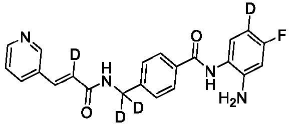 | 395.1 |
| **64** | 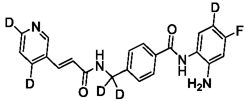 | 396.2 | **65** | 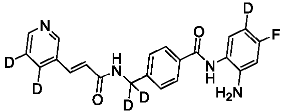 | 396.2 |
| **66** | 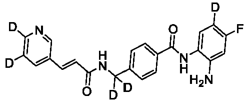 | 396.2 | **67** | 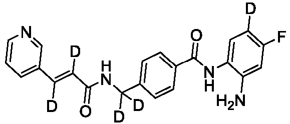 | 396.2 |
| **68** | 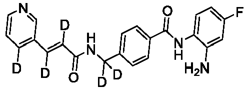 | 396.2 | **69** | 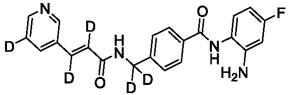 | 396.2 |
| **70** | 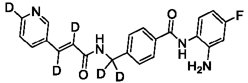 | 396.2 | **71** | 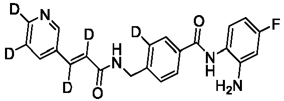 | 396.2 |
| **72** | 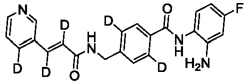 | 396.2 | **73** | 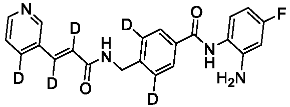 | 396.2 |
| **74** | 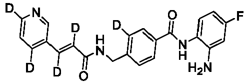 | 396.2 | **75** | 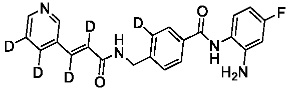 | 396.2 |
| **76** | 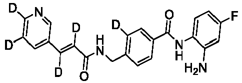 | 396.2 | **77** | 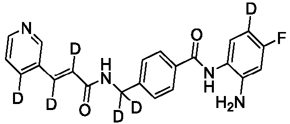 | 397.2 |
| **78** | 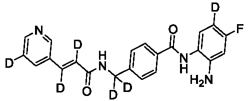 | 397.2 | **79** | 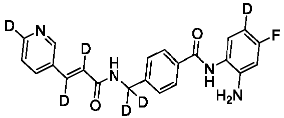 | 397.2 |
| **80** | 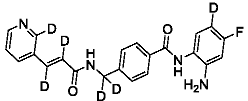 | 397.2 | **81** | 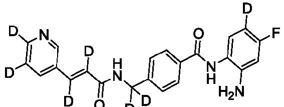 | 398.2 |
| **82** | 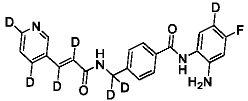 | 398.2 | **83** | 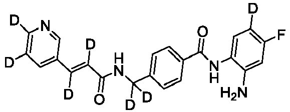 | 398.2 |
| **84** | 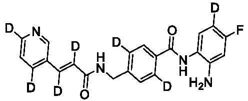 | 398.2 | **85** | 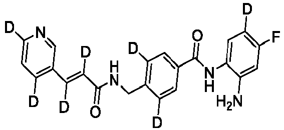 | 398.2 |
| **86** | 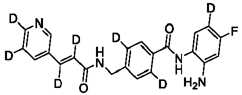 | 398.2 | **87** | 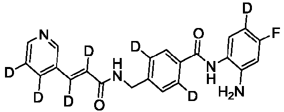 | 398.2 |

Nuclear magnetic resonance data of some of the compounds of the present disclosure are listed in Table 7.

**Tabel 7**

| **Compound** | ¹H NMR |
|---|---|
| **1** | ¹H NMR (400 MHz, DMSO-d₆) δ 9.56 (s, 1H), 8.77 (d, J = 6.3 Hz, 2H), 8.54 (d, J = 4.7 Hz, 1H), 7.99 (d, J = 8.0 Hz, 1H), 7.93 (d, J = 7.8 Hz, 2H), 7.51 (d, J = 15.9 Hz, 1H), 7.41 (dd, J = 16.0, 10.3 Hz, 3H), 7.09 (d, J = 6.3 Hz, 1H), 6.81 (d, J = 15.9 Hz, 1H), 6.52 (d, J = 11.3 Hz, 1H), 5.20 (s, 2H), 4.47 (d, J = 5.9 Hz, 2H). |
| **2** | ¹H NMR (400 MHz, DMSO-d₆) δ 9.56 (s, 1H), 8.77 (d, J *=* 8.6 Hz, 2H), 8.54 (d, J = 4.7 Hz, 1H), 7.99 (d, J = 7.9 Hz, 1H), 7.93 (d, J = 7.8 Hz, 2H), 7.51 (d, J = 15.8 Hz, 1H), 7.48 - 7.30 (m, 3H), 7.09 (t, J = 7.6 Hz, 1H), 6.81 (d, J = 15.9 Hz, 1H), 6.34 (t, J = 8.5 Hz, 1H), 5.20 (s, 2H), 4.47 (d, J = 5.9 Hz, 2H). |
| **3** | ¹H NMR (400 MHz, DMSO-d₆) δ 9.56 (s, 1H), 8.79 (t, J = 6.0 Hz, 1H), 8.76 (d, J = 2.2 Hz, 1H), 8.54 (dd, J = 4.7, 1.6 Hz, 1H), 7.99 (dt, J = 7.9, 1.9 Hz, 1H), 7.93 (d, J = 8.0 Hz, 2H), 7.51 (d, J = 15.9 Hz, 1H), 7.44 (dd, J = 8.0, 4.8 Hz, 1H), 7.39 (d, J = 8.1 Hz, 2H), 6.80 (d, J = 15.9 Hz, 1H), 6.52 (dd, J = 11.3, 2.9 Hz, 1H), 6.33 (dd, J = 8.4, 2.9 Hz, 1H), 5.22 (s, 2H), 4.47 (d, J = 6.0 Hz, 2H). |
| **4** | ¹H NMR (400 MHz, DMSO-d₆)δ 9.56 (s, 1H), 8.76 (d, *J* = 2.2 Hz, 2H), 8.54 (dd, *J* = 4.8, 1.6 Hz, 1H), 7.99 (dt, *J =* 8.0, 2.0 Hz, 1H), 7.93 (d, *J* = 8.4 Hz, 1H), 7.51 (d, *J* = 15.9 Hz, 1H), 7.46 - 7.36 (m, 3H), 7.09 (dd, *J =* 8.7, 6.3 Hz, 1H), 6.80 (d, *J* = 15.9 Hz, 1H), 6.52 (dd, *J =* 11.2, 2.9 Hz, 1H), 6.34 (td, *J =* 8.5, 2.9 Hz, 1H), 5.21 (s, 2H), 4.46 (t, *J =* 6.7 Hz, 2H). |
| **5** | ¹H NMR (400 MHz, DMSO-d₆)δ 9.56 (s, 1H), 8.82 - 8.72 (m, 2H), 8.54 (dd, *J* = 4.7, 1.6 Hz, 1H), 7.99 (dt, *J =* 8.0, 1.9 Hz, 1H), 7.96 - 7.88 (m, 2H), 7.51 (d, *J* = 15.9 Hz, 1H), 7.47 - 7.36 (m, 2H), 7.09 (dd, *J =* 8.7, 6.3 Hz, 1H), 6.80 (d, *J =* 15.9 Hz, 1H), 6.52 (dd, *J* = 11.2, 2.9 Hz, 1H), 6.34 (td, *J =* 8.5, 2.9 Hz, 1H), 5.21 (s, 2H), 4.47 (d, *J =* 6.0 Hz, 2H). |
| **8** | ¹H NMR (400 MHz, DMSO-d₆)δ 9.56 (s, 1H), 8.82 - 8.73 (m, 2H), 8.54 (dd, *J* = 4.8, 1.6 Hz, 1H), 7.99 (dq, *J* = 7.9, 1.3 Hz, 1H), 7.93 (d, *J =* 8.2 Hz, 2H), 7.48 - 7.35 (m, 3H), 7.09 (dd, *J =* 8.7, 6.3 Hz, 1H), 6.80 (s, 1H), 6.52 (dd, *J =* 11.3, 2.9 Hz, 1H), 6.34 (td, *J =* 8.5, 2.9 Hz, 1H), 5.21 (s, 2H), 4.47 (d, *J =* 6.0 Hz, 2H). |
| **9** | ¹H NMR (400 MHz, DMSO-d₆)δ 9.56 (s, 1H), 8.78 (t, *J* = 6.1 Hz, 1H), 8.54 (dd, *J* = 4.8, 1.6 Hz, 1H), 7.99 (dd, *J* = 8.0, 1.6 Hz, 1H), 7.93 (d, *J =* 8.0 Hz, 2H), 7.51 (d, *J=* 15.9 Hz, 1H), 7.46 - 7.35 (m, 3H), 7.09 (dd, *J =* 8.6, 6.3 Hz, 1H), 6.81 (d, *J =* 15.9 Hz, 1H), 6.52 (dd, *J =* 11.3, 2.9 Hz, 1H), 6.34 (td, *J =* 8.5, 2.9 Hz, 1H), 5.21 (s, 2H), 4.47 (d, *J =* 6.0 Hz, 2H). |
| **11** | ¹H NMR (400 MHz, DMSO-d₆)δ 9.56 (s, 1H), 8.83 - 8.74 (m, 2H), 8.54 (d, *J =* 1.6 Hz, 1H), 7.99 (t, *J* = 2.1 Hz, 1H), 7.93 (d, *J =* 7.9 Hz, 2H), 7.51 (d, *J =* 15.9 Hz, 1H), 7.39 (d, *J* = 8.0 Hz, 2H), 7.09 (dd, *J* = 8.7, 6.3 Hz, 1H), 6.80 (d, *J* = 15.9 Hz, 1H), 6.52 (dd, *J =* 11.2, 2.9 Hz, 1H), 6.34 (td, *J =* 8.5, 2.9 Hz, 1H), 5.21 (s, 2H), 4.47 (d, *J =* 6.0 Hz, 2H). |
| **12** | ¹H NMR (400 MHz, DMSO-d₆)δ 9.56 (s, 1H), 8.82 - 8.73 (m, 2H), 7.99 (dd, *J* = 8.0, 2.3 Hz, 1H), 7.93 (d, *J =* 8.0 Hz, 2H), 7.51 (d, *J =* 15.9 Hz, 1H), 7.41 (dd, *J* = 16.3, 8.0 Hz, 3H), 7.09 (dd, *J =* 8.7, 6.3 Hz, 1H), 6.81 (d, *J =* 15.9 Hz, 1H), 6.52 (dd, *J =* 11.2, 2.9 Hz, 1H), 6.34 (td, *J =* 8.5, 2.9 Hz, 1H), 5.21 (s, 2H), 4.47 (d, *J* = 6.0 Hz, 2H). |

### Biological Example 1. Assay for Inhibition of Jurkat and 293T Cell Proliferation by Compounds of the Present Disclosure

Jurkat or 293T cells were seeded into a 96-well plate at 3000 cells/well. After overnight adherence culture, the serially diluted compound in DMSO was added. After 72 h, the intracellular ATP level was determined by CTG. The IC₅₀ for the inhibition of cell proliferation by the compounds was calculated compared to the DMSO group. The results are shown in Table 8 below.

**Table 8. Inhibitory activity of compounds of the present disclosure against Jurkat/293T cell proliferation (IC₅₀, µM)**

| **Compound** | **Jurkat cell** | **293T cell** | **Compound** | **Jurkat cell** | **293T cell** | **Compound** | **Jurkat cell** | **293T cell** |
|---|---|---|---|---|---|---|---|---|
| **1** | 0.86 | >10 | **2** | 1.68 | >10 | **3** | 1.86 | 7.50 |
| **4** | 1.70 | 3.26 | **5** | 0.59 | >10 | **8** | 1.58 | 4.01 |
| **9** | 1.08 | 5.18 | **11** | 0.95 | 5.16 | **12** | 1.59 | 5.31 |
| **Chidamide** | 1.62 | 6.71 | | | | | | |

As can be seen from the data in the table above, the compounds of the present disclosure selectively killed Jurkat cells, and exhibited a marked difference in cytotoxicity between human embryonic kidney cells 293T and tumor cells Jurkat. Compound 1 and compound 5 exhibited stronger cytotoxicity against tumor cells Jurkat and weaker cytotoxicity against 293T cells compared to chidamide.

### Biological Example 2. Determination of Intracellular Acetyl Lysine/H3K 27 Acetyl Lysine Level with Compound of the Present Disclosure

HeLa cells were seeded into a 96-well plate at 20000 cells/well. After overnight adherence culture, the serially diluted compound was added. After 24 h of treatment, intracellular levels of acetyl lysine and H3K 27 acetyl lysine were quantified by ELISA. The results are shown in Table 9 below.

**Table 9. Effect of compound of the present disclosure on levels of acetyl lysine and H3K 27 acetyl lysine**

| **Compound** | **Acetyl lysine** | | **H3K 27 Acetyl lysine** | |
|---|---|---|---|---|
| | Minimum effective concentration | Maximum (%) | Minimum effective concentration | Maximum (%) |
| **1** | 16 nM | 430% | 80 nM | 360% |
| **2** | 16nM | 420% | 80nM | 360% |
| **5** | 16nM | 350% | 80nM | 420% |
| **11** | 80nM | 450% | 80nM | 460% |
| **Chidamide** | 80nM | 420% | 80nM | 380% |

### Biological Example 3. Assay for Inhibitory Activity of Compound of the Present Disclosure Against HDAC Enzyme

The effect of compound 5 of the present disclosure on the activity of HDAC enzymes was determined using a fluorometric assay. Serially diluted DMSO sample solutions were added to the reaction wells, enzymes were added to a 384-well plate, and a reaction buffer was added to the control wells. The plate was incubated at room temperature for 15 min. After the fluorogenic substrate solution was added, the reaction was started. Fluorescence intensity readings (excitation: 355 nM, emission: 460 nM) were recorded every minute for 60 min using a Paradigm detector, and the slope values were calculated. Inhibition rate (%) = (maximum value - sample value)/(maximum value - minimum value) × 100%. The IC₅₀ values were obtained by fitting a curve using the equation: Y = Bottom + (Top - Bottom)/(1 + (IC₅₀/X)^HillSlope), where Y represents the inhibition rate and X represents the concentration of the compound. The results are shown in Table 10 below.

**Table 10. Inhibitory activity of compound of the present disclosure against HDAC1, HDAC2, HDAC3, and HDAC10 (IC₅₀, nM)**

| **Compound** | **HDAC1** | **HDAC2** | **HDAC3** | **HDAC10** | **HDAC6** |
|---|---|---|---|---|---|
| **5** | 67 | 153 | 657 | 127 | >30000 |
| **Chidamide** | 80 | 128 | 583 | 318 | >30000 |

The results showed that compound 5 of the present disclosure exhibited relatively good inhibitory activity against four subtypes HDAC1, HDAC2, HDAC3, and HDAC10, where the activity against HDAC1 was slightly stronger than that of chidamide (1.2-fold), the activity against HDAC2 was slightly weaker than that of chidamide (0.83-fold), and the activity against HDAC10 was 2.5 times that of chidamide. The IC₅₀ values of inhibitory activity against HDAC6 by Compound 5 and Chidamide both exceeded 30 mM.

### Biological Example 4. NK Cell-Mediated Tumor Cell Killing by Compounds of the Present Disclosure

OVCAR 3 cells or NK92 cells expressing GFP were seeded into a 96-well plate at 4000 cells/well. After overnight adherence culture, the serially diluted compounds were added, and the plate was incubated for another 72 h. After the two types of cells were mixed and cultured for another 1-4 h, NK92 cells were removed by washing with PBS, and adherent GFP-positive tumor cells were counted. The EC₅₀ of the compounds in promoting the NK92 cell-mediated OVCAR 3 cell death was calculated compared to the DMSO group. The results are shown in Table 11 below.

**Table 11. NK cell-mediated tumor cell killing by compounds of the present disclosure**

| **Compound** | EC₅₀ (nM) | **Compound** | EC₅₀ (nM) | **Compound** | EC₅₀ (nM) |
|---|---|---|---|---|---|
| **1** | 43.9 | 5 | 33.7 | **Chidamide** | 161 |

The results showed that compound 1 and compound 5 of the present disclosure exhibited a significantly superior effect on NK cell-mediated tumor cell killing to that of the control drug chidamide.

### Biological Example 5. Assay for Stability of Compounds of the Present Disclosure in Liver Microsomes

After the 1 µM compound was incubated with 0.5 mg/mL of human or mouse liver microsomes and an NADPH regeneration system at 37 °C for different times, respectively, LC-MS-MS was used to analyze the remaining amount of the compound and calculate T_{1/2}. The results are shown in Table 12 below.

**Table 12. Evaluation results of stability of compounds of the present disclosure in human and mouse liver microsomes**

| **Compound** | **Species** | **T_{1/2} (min)** | **Clᵢₙₜ (mL/min/kg)** | **Clₗᵢᵥₑᵣ (mL/min/kg)** |
|---|---|---|---|---|
| **4** | Human | 89.34 | 19.46 | 10.03 |
| | Mouse | 22.38 | 243.95 | 65.74 |
| **5** | Human | 218.69 | 7.95 | 5.74 |
| | Mouse | 23.60 | 231.26 | 64.79 |
| **8** | Human | 94.73 | 18.35 | 9.73 |
| | Mouse | 19.14 | 285.16 | 68.41 |
| **9** | Human | 379.06 | 4.59 | 3.75 |
| | Mouse | 46.41 | 117.62 | 50.99 |
| **11** | Human | 73.43 | 23.68 | 11.04 |
| | Mouse | 23.64 | 230.94 | 64.76 |
| **12** | Human | 605.14 | 2.87 | 2.52 |
| | Mouse | 50.63 | 107.81 | 49.05 |
| **Chidamide** | Human | 83.03 | 20.94 | 10.41 |
| | Mouse | 24.59 | 222.01 | 64.04 |

As can be seen from the data in the table above, the stability of some of the compounds of the present disclosure in human liver microsomes was significantly superior to that of chidamide.

### Biological Example 6. Identification Assay for Metabolites of Compounds of the Present Disclosure in Liver Microsomes and Hepatocytes from Various Species

After the 10 µM compound was separately incubated with 1.0 mg/mL liver microsomes or hepatocytes (from 5 species: mouse, rat, dog, monkey, and human), and an NADPH regeneration system at 37 °C for 120 min, a stop solution was added to terminate the reaction, and the incubated samples were removed from the water bath. After the reaction was terminated, the samples were vortexed and then centrifuged at 4 °C and 3260× g for 15 min. After centrifugation, all the supernatants were transferred to a 96-well plate for direct use in LC-MS analysis, or subjected to dilution or concentration and reconstitution prior to injection. An LC-UV-HRMSn (n = 1-2) analytical method was established on LC-UV-HRMSn, and data acquisition was performed using SCIEX OS software. The parent drug and the metabolites could be analyzed by mass spectrometry with different scanning modes (MS/MS or IDA) and ultraviolet full-wavelength scanning (λ = 190-500 nm). By comparing the ultraviolet and mass spectra of the post-incubation samples with those of blank samples from the same species, potential metabolites were identified. The possible structures of the metabolites were inferred by comparative analysis of the collision-induced dissociation (CID) fragments of the test compounds and their metabolites. The metabolites and their relative abundances were summarized in a table for interspecies comparison.

The experimental results showed that some of the compounds of the present disclosure, particularly compound 5, exhibited simple metabolic pathways and good stability in liver microsomes from all the species.

### LC-MS analysis conditions:

| | |
|---|---|
| LC system: | AB Sciex ExionLC |
| PDA detector | PDA, λ: 190 - 500 nm |
| Mass spectrometry: | Zeno TOF 7600 |
| Column: | ACQUITY UPLC^{®} HSS T3, 1.8 µm, 2.1×100 mm |
| Column temperature: | 40°C |
| Auto-sampler: | 10°C |
| Mobile phase: | A: 0.1% FA in Water; B: 0.1% FA in ACN |
| Flow rate: | 0.5 mL/min |

### Elution gradient:

| **Step** | **Time (min)** | **A%** | **B%** |
|---|---|---|---|
| **1** | 0.00 | 95.0 | 5.0 |
| **2** | 2.00 | 95.0 | 5.0 |
| **3** | 3.00 | 80.0 | 20.0 |
| **4** | 12.00 | 68.0 | 32.0 |
| **5** | 14.00 | 10.0 | 90.0 |
| **6** | 18.00 | 10.0 | 90.0 |
| **7** | 18.01 | 95.0 | 5.0 |
| **8** | 21.00 | 95.0 | 5.0 |

### LC-MS/MS analysis conditions:

| | |
|---|---|
| LC system: | AB Sciex ExionLC |
| PDA detector | PDA, λ: 190 - 500 nm |

| | |
|---|---|
| Mass spectrometry: | Zeno TOF 7600 |
| Column: | ACQUITY UPLC^{®} HSS T3, 1.8 µm, 2.1×100 mm |
| Column temperature: | 40°C |
| Auto-sampler: | 10°C |
| Mobile phase: | A: 0.1% FA in Water; B: 0.1% FA in ACN |
| Flow rate: | 0.5 mL/min |
| Injection volume: | 3 µL |

### Elution gradient:

| **Step** | **Time (min)** | **A%** | **B%** |
|---|---|---|---|
| **1** | 0.00 | 95.0 | 5.0 |
| **2** | 2.00 | 95.0 | 5.0 |
| **3** | 3.00 | 80.0 | 20.0 |
| **4** | 12.00 | 68.0 | 32.0 |
| **5** | 14.00 | 10.0 | 90.0 |
| **6** | 18.00 | 10.0 | 90.0 |
| **7** | 18.01 | 95.0 | 5.0 |
| **8** | 21.00 | 95.0 | 5.0 |

### Mass spectrometry parameters:

| **Mass Spectrometry Parameters** | **Value** |
|---|---|
| Ionization Mode: | ESI⁺ |
| Ion Spray Voltage(V): | 5500 |
| Source Temperature(°C): | 550 |
| CAD gas: | 8 |
| Gas 1 (psi): | 55 |
| Gas 2 (psi): | 55 |
| CE(V): | 25±15 |
| DP (V): | 80 |
| Mass Range | 50-1200 |
| Curtain gas | 35 |
| Scan Mode: | IDA |

### Biological Example 7. Pharmacokinetic Experiment of Compounds of the Present Disclosure in Mice

CD-1 female mice aged 7 to 10 weeks were intravenously administered and orally administered at doses of 1 mg/kg and 10 mg/kg, respectively. The mice were fasted for at least 12 h before the administration and given food 4 h after the administration, and they were given *ad libitum* access to water during the experiment.

On the day of the experiment, animals in the intravenous group were given the corresponding compound by single injection via the tail vein at an administration volume of 10 mL/kg; and animals in the oral group were given the corresponding compound by single intragastric injection at an administration volume of 10 mL/kg. The animals were weighed before administration, and the administration volume was calculated according to the body weight. The sample collection time points were 0.083 h, 0.167 h, 0.5 h, 1 h, 2 h, 4 h, 8 h, and 24 h. About 200 µL of whole blood was collected through the orbital venous plexus at each time point and used to prepare plasma for concentration determination by high-performance liquid chromatography-tandem mass spectrometry (LC-MS/MS). The plasma concentrations were processed using a non-compartmental model of Winnolin pharmacokinetic software, and the pharmacokinetic parameters were calculated using a linear-log trapezoidal method. The results are shown in Table 13 below.

**Table 13. Results of in vivo pharmacokinetic evaluation of compounds of the present disclosure**

| **Route of Administration** | **Pharmacokinetic parameter** | **Chidamide** | **Compound 1** | **Compound 2** | **Compound 3** | **Compound 4** |
|---|---|---|---|---|---|---|
| Injection (1 mg/kg) | Vdss (L/Kg) | 1.55 | 2.19 | 1.61 | 0.93 | 1.32 |
| | T_{1/2} (h) | 2.03 | 2.41 | 2.30 | 2.78 | 3.05 |
| | Cl (mL/min/Kg) | 33.88 | 41.54 | 37.81 | 28.13 | 27.68 |
| | AUC₀₋ₗₐₛₜ (h*ng/mL) | 538.94 | 401.19 | 440.78 | 592.45 | 601.93 |
| Oral administratio n (10 mg/kg) | Cₘₐₓ (ng/mL) | 3746.67 | 3453.33 | 2363.42 | 2350 | 1736.67 |
| | Tₘₐₓ (h) | 0.17 | 0.08 | 0.08 | 0.08 | 0.08 |
| | T_{1/2} (h) | 3.51 | 2.76 | 1.85 | 2.21 | 3.90 |
| | AUC₀₋ₗₐₛₜ (h*ng/mL) | 2487.02 | 1889.97 | 2163.42 | 1768.48 | 1713.91 |
| | F% | 46.13% | 45.33% | 46.54% | 26.34% | 28.47% |

| **Route of Administration** | **Pharmacokinetic parameter** | **Compound 5** | **Compound 8** | **Compound 9** | **Compound 11** | **Compound 12** |
|---|---|---|---|---|---|---|
| Injection (1 mg/kg) | Vdss (L/Kg) | 1.13 | 1.21 | 0.44 | 0.62 | 0.71 |
| | T_{1/2} (h) | 1.28 | 1.01 | 1.48 | 1.79 | 1.17 |
| | Cl (mL/min/Kg) | 36.76 | 36.90 | 16.63 | 22.72 | 32.44 |
| | AUC₀₋ₗₐₛₜ (h*ng/mL) | 453.33 | 903.35 | 1001.97 | 733.34 | 513.67 |
| Oral administratio n (10 mg/kg) | Cₘₐₓ (ng/mL) | 1330.33 | 824.33 | 1639.33 | 2373.33 | 2080 |
| | Tₘₐₓ (h) | 0.17 | 0.08 | 0.17 | 0.08 | 0.17 |
| | T_{1/2} (h) | 3.13 | 1.87 | 1.21 | 1.19 | 1.93 |
| | AUC₀₋ₗₐₛₜ (h*ng/mL) | 2356.13 | 1433.57 | 1052.22 | 1329.57 | 1607.37 |
| | F% | 51.97% | 29.56% | 10.49% | 18.19% | 30.48% |

As can be seen from the data in the table above, some of the compounds of the present disclosure exhibited significant differences in pharmacokinetics (PK) in mice, indicating that deuterium substitution at different positions had a significant effect on the PK of the compounds. Among them, the bioavailability of compound 5 in mice was superior to that of chidamide.

### Biological Example 8. Pharmacokinetic Experiment of Compounds of the Present Disclosure in Rats

Healthy female rats aged 6 to 8 weeks were intravenously administered and orally administered at doses of 1 mg/kg and 10 mg/kg. The rats were fasted overnight before the administration.

On the day of the experiment, animals in the intravenous group were given the corresponding compound by single injection via the tail vein at an administration volume of 10 mL/kg; and animals in the oral group were given the corresponding compound by single intragastric injection at an administration volume of 10 mL/kg. The sample collection time points were 0.083 h, 0.167 h, 0.5 h, 1 h, 2 h, 4 h, 8 h, and 24 h. About 200 µL of whole blood was collected through the neck at each time point and used to prepare plasma for concentration determination by high-performance liquid chromatography-tandem mass spectrometry (LC-MS/MS). The plasma concentrations were processed using a non-compartmental model of Winnolin pharmacokinetic software, and the pharmacokinetic parameters were calculated using a linear-log trapezoidal method. The results are shown in Table 14 below.

**Table 14. Results of in vivo pharmacokinetic evaluation of compounds**

| **Route of Administration** | **Pharmacokinetic parameter** | **Compound 5** |
|---|---|---|
| Injection (1 mg/kg) | Vdss (L/Kg) | 2.42 |
| | T_{1/2} (h) | 6.54 |
| | Cl (mL/min/Kg) | 12.92 |
| | AUC₀₋ₗₐₛₜ (h*ng/mL) | 1255.18 |
| Oral administration (10 mg/kg) | Cₘₐₓ (ng/mL) | 3423.33 |
| | Tₘₐₓ (h) | 0.16 |
| | T_{1/2} (h) | 4.01 |
| | AUC₀₋ₗₐₛₜ (h*ng/mL) | 5147.83 |
| | F% | 40.14% |

### Biological Example 9. In Vivo Efficacy Experiment of Compounds of the Present Disclosure in MC-38 Model

Female C57BL6N mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. Mouse colon cancer MC38 cells were cultured conventionally in 1640 medium containing 10% fetal bovine serum in an incubator at 37 °C with 5% CO₂. After being subcultured, the cells were collected when they reached the desired amount. The C57BL6N mice were subcutaneously injected with 2 × 10⁶ MC38 cells on the right side to form tumors. After the tumors grew to about 100 mm³, the animals were randomly grouped for administration. Tumor volumes were measured with a caliper on day 3, day 7, day 10, day 14, day 17, and day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(tumor volume on day 28 in control group - tumor volume on day 1 in control group). The toxicity of the compounds was evaluated according to the body weight and state of the mice.

The groups were as follows:
1) solvent control group; 2) PD-1 group; 3) BDO group; 4) chidamide group; 5) compound 1 group; 6) compound 5 group; 7) PD-1 + BDO group; 8) compound 1 and PD-1 combination group; 9) compound 1 and BDO combination group; 10) compound 5 and PD-1 combination group; 11) compound 5 and BDO combination group; 12) compound 1 and PD-1 and BDO combination group; 13) compound 5 and PD-1 and BDO combination group; 14) chidamide and PD-1 and BDO combination group, with 6 mice per group. The results are shown in Table 15 below.

**Table 15. In vivo efficacy of some of the compounds of the present disclosure in MC-38 model**

| **Grou p** | **Compound** | **Dose** | **Administrati on period** | **Route of Administration** | **TGI( %)** | **Change in body weight** |
|---|---|---|---|---|---|---|
| **1** | Solvent control group | -- | QD (21 days) | PO | -- | +23.0% |
| **2** | PD-1 | 1mg/kg | QW(21 days) | IV | 51.8% | +14.1% |
| **3** | BDO | 2mg/kg | QW(21 days) | IP | 63.9% | +14.8% |
| **4** | Chidamide | 15mg/kg | QD(21 days) | PO | 8.9% | +21.1% |
| **5** | Compound 1 | 15mg/kg | QD(21 days) | PO | 35.7% | +17.6% |
| **6** | Compound 5 | 15mg/kg | QD(21 days) | PO | 12.2% | +24.2% |
| **7** | PD-1 +BDO | 1mg/kg +2mg/kg | QW +QW(21 days) | IV +IP | 55.3% | +10.3% |
| **8** | Compound 1 +PD-1 | 15mg/kg +1mg/kg | QD +QW(21 days) | PO +IV | 82.3% (1PR+ 1SD) | +9.3% |
| **9** | Compound 1 +BDO | 15mg/kg +2mg/kg | QD +QW(21 days) | PO +IP | 77.3% | +9.0% |
| **10** | Compound 5 +PD-1 | 15mg/kg +1mg/kg | QD +QW(21 days) | PO +IV | 81.3% (2SD) | +8.5% |
| **11** | Compound 5 +BDO | 15mg/kg +2mg/kg | QD +QW(21 days) | PO +IP | 72.3% | +9.1% |
| **12** | Compound 1 +PD-1 +BDO | 15mg/kg +1mg/kg +2mg/kg | QD +QW +QW(21 days) | PO +IV +IP | 84.7% (2PR) | +5.5% |
| **13** | Compound 5 +PD-1+BDO | 15mg/kg +1mg/kg+2mg/kg | QD +QW +QW(21 days) | PO +IV +IP | 87.2% (3PR) | +5.3% |
| **14** | Chidamide +PD-1 +BDO | 15mg/kg +1mg/kg +2mg/kg | QD +QW +QW(21 days) | PO +IV +IP | 85.7% (1PR) | +6.6% |

| | | | | | | |
|---|---|---|---|---|---|---|
| Note: BDO represents an anti-VEGF antibody; PD-1 represents an anti-PD-1 antibody; IV represents administration via intravenous injection; IP represents administration via intraperitoneal injection; PO represents oral administration; QD represents once-daily administration; QW represents once-weekly administration; PR represents partial response (tumor volume reduction exceeding 30% relative to the initial volume upon completion of the administration cycle); SD represents stable disease (tumor volume reduction or growth not exceeding 30% relative to the initial volume upon completion of the administration cycle). As can be seen from the results of the *in vivo* experiment described above, the compounds of the present disclosure, when administered in combination with PD-1 and BDO, exhibited a relatively good inhibitory effect on the MC-38 *in vivo* tumor model. Among them, compound 5 + PD-1 + BDO (group 13) was able to cause tumor regression in 50% (3/6) of mice, and the effect was significantly superior to that of chidamide + PD-1 + BDO (group 14, 1/6). | | | | | | |

### Biological Example 10. In Vivo Efficacy Experiment of Compounds of the Present Disclosure in CT-26 Model

Female BALB/c mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. Mouse colon cancer CT-26 cells were cultured conventionally in 1640 medium containing 10% fetal bovine serum in an incubator at 37 °C with 5% CO₂. After being subcultured, the cells were collected when they reached the desired amount. The BALB/c mice were subcutaneously injected with 2 × 10⁵ CT-26 cells on the right side to form tumors. After the tumors grew to about 100 mm³, the animals were randomly grouped for administration. Tumor volumes were measured with a caliper on day 3, day 7, day 10, day 14, day 17, and day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(tumor volume on day 28 in control group - tumor volume on day 1 in control group). The toxicity of the compounds was evaluated according to the body weight and state of the mice.

The groups were as follows:
1) solvent control group; 2) PD-1 group; 3) BDO group; 4) chidamide group; 5) compound 1 group; 6) compound 5 group; 7) PD-1 + BDO group; 8) compound 1 and PD-1 combination group; 9) compound 1 and BDO combination group; 10) compound 5 and PD-1 combination group; 11) compound 5 and BDO combination group; 12) compound 1 and PD-1 and BDO combination group; 13) compound 5 and PD-1 and BDO combination group; 14) chidamide and PD-1 and BDO combination group, with 6 mice per group. The results are shown in Table 16 below.

**Table 16. In vivo efficacy of some of the compounds of the present disclosure in CT-26 model**

| **Group** | **Compound** | **Dose** | **Administration period** | **Route of Administration** | **TGI(%)** | **Change in body weight** |
|---|---|---|---|---|---|---|
| **1** | Solvent control group | -- | QD (21 days) | PO | -- | +23.0% |
| **2** | PD-1 | 1mg/kg | QW(21 days) | IV | 51.8% | +14.1% |
| **3** | BDO | 2mg/kg | QW(21 days) | IP | 63.9% | +14.8% |
| **4** | Chidamide | 15mg/kg | QD(21 days) | PO | 8.9% | +21.1% |
| **5** | Compound 1 | 15mg/kg | QD(21 days) | PO | 35.7% | +17.6% |
| **6** | Compound 5 | 15mg/kg | QD(21 days) | PO | 12.2% | +24.2% |
| **7** | PD-1 +BDO | 1mg/kg +2mg/kg | QW +QW(21 days) | IV +IP | 55.3% | +10.3% |
| **8** | Compound 1 +PD-1 | 15mg/kg +1mg/kg | QD +QW(21 days) | PO +IV | 82.3% (1PR+1 SD) | +9.3% |
| **9** | Compound 1 +BDO | 15mg/kg +2mg/kg | QD +QW(21 days) | PO +IP | 77.3% | +9.0% |
| **10** | Compound 5 +PD-1 | 15mg/kg +1mg/kg | QD +QW(21 days) | PO +IV | 81.3% (2SD) | +8.5% |
| **11** | Compound 5 +BDO | 15mg/kg +2mg/kg | QD +QW(21 days) | PO +IP | 72.3% | +9.1% |
| **12** | Compound 1 +PD-1 +BDO | 15mg/kg+1mg/kg +2mg/kg | QD +QW +QW(21 days) | PO +IV +IP | 84.7% (2PR) | +5.5% |
| **13** | Compound 5 +PD-1 +BDO | 15mg/kg +1mg/kg +2mg/kg | QD +QW +QW(21 days) | PO +IV +IP | 87.2% (3PR) | +5.3% |
| **14** | Chidamide + PD-1 +BDO | 15mg/kg +1mg/kg +2mg/kg | QD +QW +QW(21 days) | PO +IV +IP | 85.7% (1PR) | +6.6% |

| | | | | | | |
|---|---|---|---|---|---|---|
| Note: BDO represents an anti-VEGF antibody; PD-1 represents an anti-PD-1 antibody; IV represents administration via intravenous injection; IP represents administration via intraperitoneal injection; PO represents oral administration; QD represents once-daily administration; QW represents once-weekly administration; PR represents partial response (tumor volume reduction exceeding 30% relative to the initial volume upon completion of the administration cycle); SD represents stable disease (tumor volume reduction or growth not exceeding 30% relative to the initial volume upon completion of the administration cycle). CR represents complete regression of the tumor. | | | | | | |

As can be seen from the results of the *in vivo* experiment described above, the compounds of the present disclosure, when administered in combination with PD-1 and BDO, exhibited a relatively good inhibitory effect on the CT-26 *in vivo* tumor model. Among them, compound 5 + PD-1 + BDO (group 13) was able to cause tumor regression or slow the growth of tumors in (5/6) mice, and the effect was significantly superior to that of chidamide + PD-1 + BDO (group 14, 2/6).

Although specific embodiments of the present disclosure have been described above, it will be appreciated by those skilled in the art that these embodiments are merely illustrative and that many changes or modifications can be made to these embodiments without departing from the principles and spirit of the present disclosure. The protection scope of the present disclosure is therefore defined by the appended claims.

## Claims

1. A compound of general formula (1) or an isomer thereof, a crystalline form thereof, a pharmaceutically acceptable salt thereof, a hydrate thereof, or a solvate thereof: 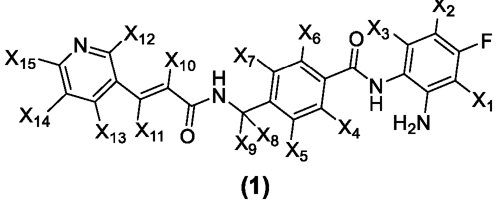 wherein in general formula (1):
X₁, X₂, X₃, X₄, X₅, X₆, X₇, X₈, X₉, X₁₀, X₁₁, X₁₂, X₁₃, X₁₄, and X₁₅ are each independently hydrogen or deuterium, and at least one of X₁, X₂, X₃, X₄, X₅, X₆, X₇, X₈, X₉, X₁₀, X₁₁, X₁₂, X₁₃, X₁₄, or X₁₅ is selected from deuterium.

2. The compound or the isomer thereof, the crystalline form thereof, the pharmaceutically acceptable salt thereof, the hydrate thereof, or the solvate thereof according to claim 1, wherein in general formula (1), X₈ = X₉.

3. The compound or the isomer thereof, the crystalline form thereof, the pharmaceutically acceptable salt thereof, the hydrate thereof, or the solvate thereof according to claim 1 or 2, wherein the compound has one of the following structures:

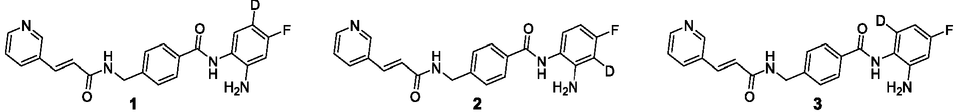

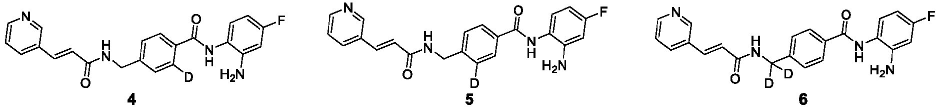

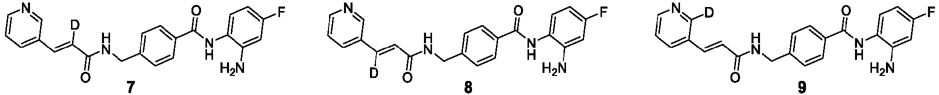

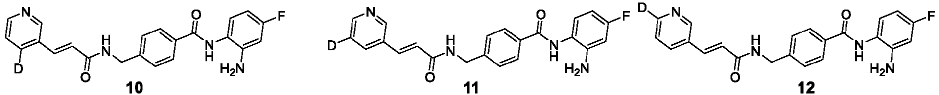

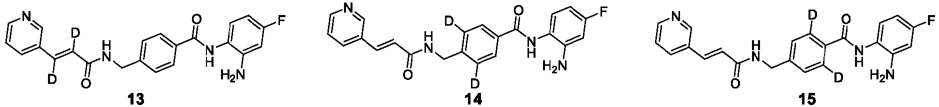

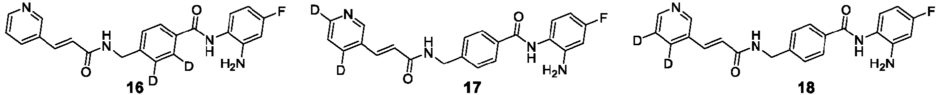

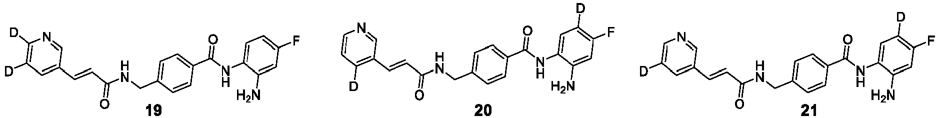

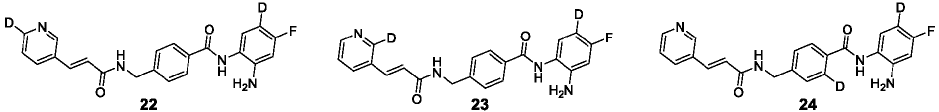

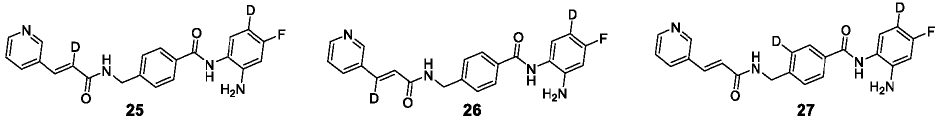

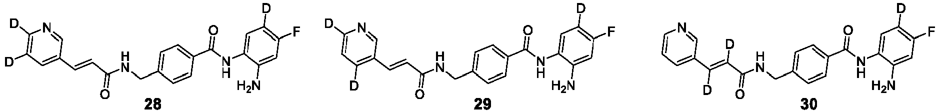

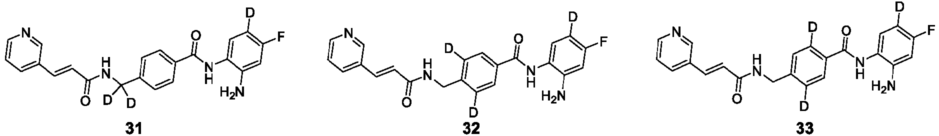

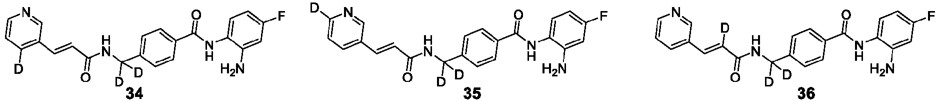

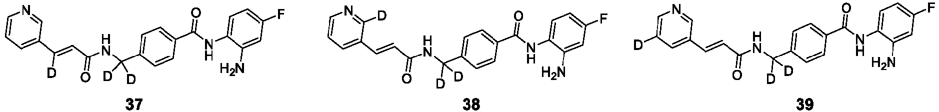

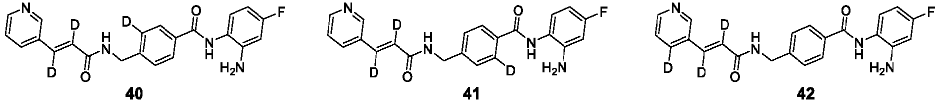

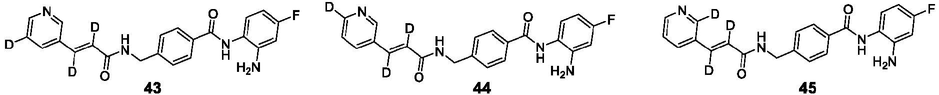

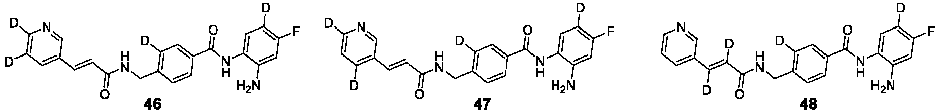

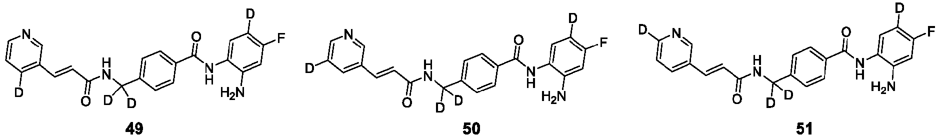

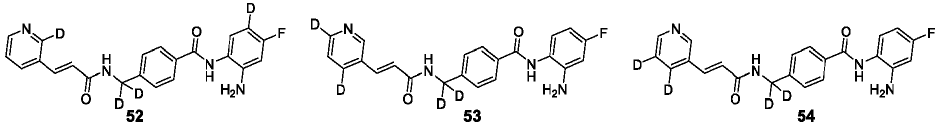

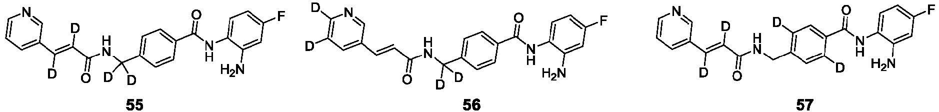

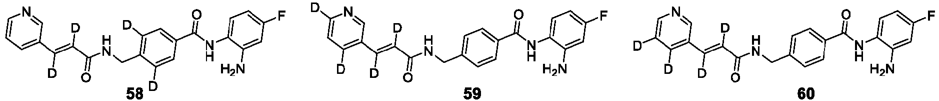

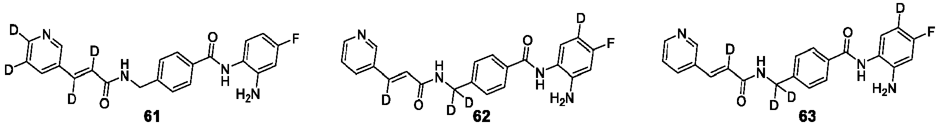

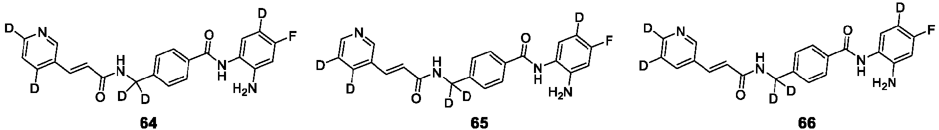

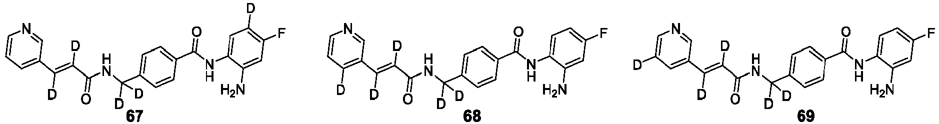

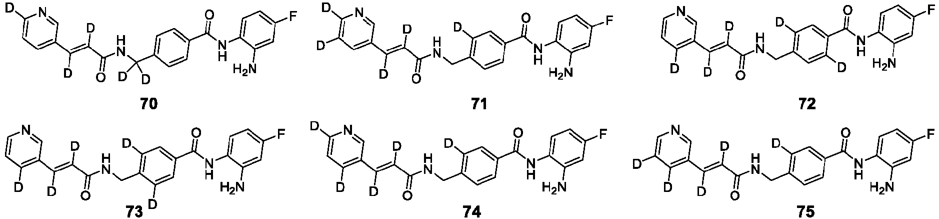

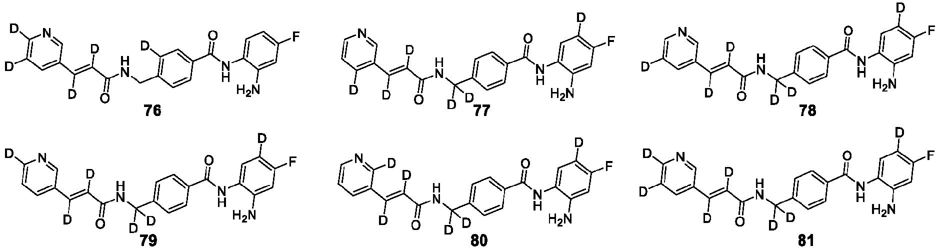

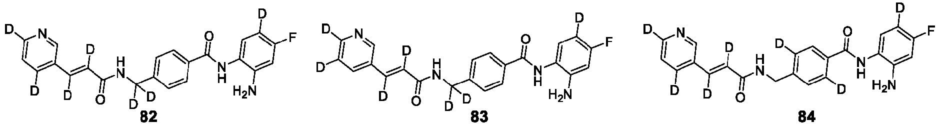

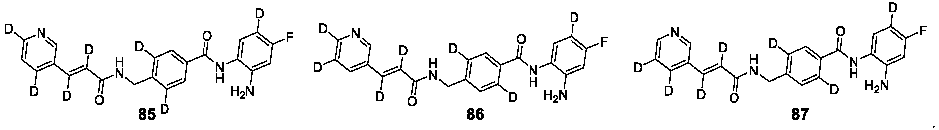

4. The compound or the isomer thereof, the crystalline form thereof, the pharmaceutically acceptable salt thereof, the hydrate thereof, or the solvate thereof according to claim 1 or 2, wherein the compound has one of the following structures:

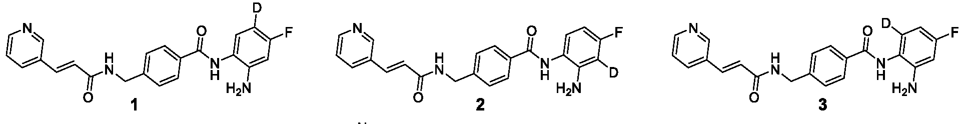

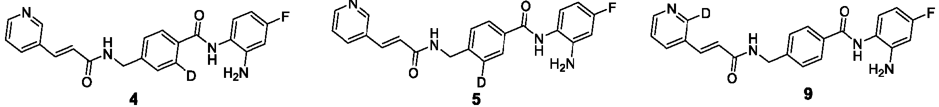

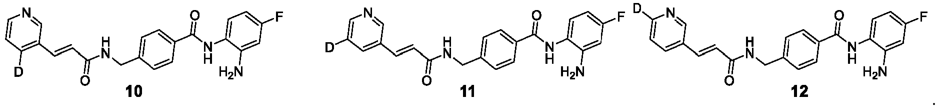

5. The compound or the isomer thereof, the crystalline form thereof, the pharmaceutically acceptable salt thereof, the hydrate thereof, or the solvate thereof according to claim 1 or 2, wherein the compound has the following structure:

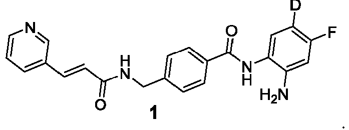

6. The compound or the isomer thereof, the crystalline form thereof, the pharmaceutically acceptable salt thereof, the hydrate thereof, or the solvate thereof according to claim 1 or 2, wherein the compound has the following structure:

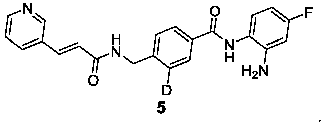

7. A pharmaceutical composition, comprising a pharmaceutically acceptable excipient or carrier, and the compound or the isomer thereof, the crystalline form thereof, the pharmaceutically acceptable salt thereof, the hydrate thereof, or the solvate thereof according to any one of claims 1 to 6 as an active ingredient.

8. A pharmaceutical composition, comprising a pharmaceutically acceptable excipient or carrier, and a therapeutically effective amount of the compound or the isomer thereof, the crystalline form thereof, the pharmaceutically acceptable salt thereof, the hydrate thereof, or the solvate thereof according to any one of claims 1 to 6, and a therapeutically effective amount of an immune checkpoint inhibitor as active ingredients.

9. A pharmaceutical composition, comprising a pharmaceutically acceptable excipient or carrier, and a therapeutically effective amount of the compound or the isomer thereof, the crystalline form thereof, the pharmaceutically acceptable salt thereof, the hydrate thereof, or the solvate thereof according to any one of claims 1 to 6, and a therapeutically effective amount of an immune checkpoint inhibitor and a therapeutically effective amount of a VEGFR inhibitor as active ingredients.

10. Use of the compound or the isomer thereof, the crystalline form thereof, the pharmaceutically acceptable salt thereof, the hydrate thereof, or the solvate thereof according to any one of claims 1 to 6 or the pharmaceutical compositions according to claims 7 to 9 in preparing a medicament for treating, regulating, and/or preventing an HDAC inhibitor-associated disease.

11. The use according to claim 10, wherein the disease is cancer, and the cancer is a hematologic cancer or a solid tumor.

12. The use according to claim 11, wherein the cancer comprises breast cancer, colon cancer, uterine cancer, pancreatic cancer, lung cancer, gastric cancer, leukemia, lymphoma, prostate cancer, liver cancer, cervical cancer, neuroblastoma, melanoma, or intracranial tumors.
